# EUROPEAN PATENT APPLICATION

(11) **EP 3 812 373 A1**
(43) Date of publication of application: **28.04.2021**
(21) Application number: 19823442.9
(22) Date of filing: 21.06.2019
(51) Int. Cl.: C07D 243/38, A61K 31/5513, A61K 51/00, A61P 25/18, A61P 25/20, A61P 25/28, A61P 43/00, C07K 14/705

(54) **NOVEL COMPOUND BINDING TO DESIGNER RECEPTOR, IMAGING METHOD FOR DESIGNER RECEPTOR, AGONIST OR ANTAGONIST, THERAPEUTIC AGENT, COMPANION DIAGNOSTIC AGENT, AND IMAGING METHOD FOR NERVE CELL**

(30) Priority: 21.06.2018 JP 2018118210
(71) Applicant: NATIONAL INSTITUTES FOR QUANTUM AND RADIOLOGICAL SCIENCE AND TECHNOLOGY, Inage-ku Chiba-shi Chiba 263-8555 (JP)
(72) Inventor: MINAMIMOTO Takafumi, Chiba-shi, Chiba 263-8555 (JP); NAGAI Yuji, Chiba-shi, Chiba 263-8555 (JP); JI Bin, Chiba-shi, Chiba 263-8555 (JP); MIYAKAWA Naohisa, Chiba-shi, Chiba 263-8555 (JP); HIGUCHI Makoto, Chiba-shi, Chiba 263-8555 (JP); SUHARA Tetsuya, Chiba-shi, Chiba 263-8555 (JP)
(74) Representative: Prüfer & Partner mbB Patentanwälte · Rechtsanwälte
(86) International application number: PCT/JP2019/024834
(87) International publication number: WO 2019/245047

(57) **Abstract**

The purpose of the present invention is to provide a technique for imaging the brain of a live animal and application of the technique. A radiolabeled dibenzoazepine derivative, which shows excellent brainparmeability, high receptor selectivity and high quantitativity, is used for imaging a live animal body. A dibenzoazepine derivative is used for treating a disease in which hM4D receptor or hM3D receptor participates. Further, a radiolabeled dibenzoazepine derivative is used for imaging an axonal end which is innervated by a nerve cell.

## Description

### TECHNICAL FIELD

The present invention relates to a technique for visualizing an hM4D receptor or an hM3D receptor artificially expressed in the brain, a technique for visualizing a nerve cell, and a therapeutic agent for a disease associated with the hM4 receptor or the hM3 receptor.

### BACKGROUND ART

Conventionally, research has been conducted on a mechanism and treatment for diseases by introducing designer receptors into the body and using agents selectively acting on the designer receptors. Receptors to be used for the research are called Designer Receptor Exclusively Activated by Designer Drug (DREADD), on which many studies have been conducted at a cellular level.
Non-Patent Document 1: Armbruster BN et al., Evolving the lock to fit the key to create a family of G protein-coupled receptors potently activated by an inert ligand. Proc Natl Acad Sci USA. Mar 20; 104(12): 5163-5168 (2007)
Non-Patent Document 2: Ji et al., Multimodal Imaging for DREADD-Expressing Neurons in Living Brain and Their Application to Implantation of iPSC-Derived Neural Progenitors. Journal Neurosci. 36(45): 11544-11558 (2016)
Non-Patent Document 3: Nagai et al., PET imaging-guided chemogenetic silencing reveals a critical role of primate rostromedial caudate in reward evaluation. Nature Communications. 7: 13605 (2016)
Non-Patent Document 4: Gomez J. et al., Chemogenetics revealed: DREADD occupancy and activation via converted clozapine, Science 357(6350): 503-507 (2017)

### DISCLOSURE OF THE INVENTION

### Problems to be Solved by the Invention

In in vivo research on the techniques, it is required to express an introduced designer receptor in a specific organ of interest or a specific region of the organ in a targeted manner. Expression of the designer receptor in vivo has been confirmed by preparing and staining a tissue specimen (e.g., a section). However, this method has a limitation on the number of individuals to be tested in the case of large animals and has a poor development efficiency in finding out a disease mechanism and developing a therapeutic agent.

Meanwhile, as another method for confirming the expression of the designer receptor in vivo, research utilizing positron emission tomography (PET) has proceeded. There is a great expectation for this method since a preparation of a tissue specimen (e.g., a section) is not needed, pharmacokinetics in vivo can be dynamically observed, and a timing, site, range, intensity, and the like of expression can be quantitatively determined.

Specifically, after a gene for expressing a designer receptor is introduced into a specific organ or a specific region and then a predetermined period required for expressing the receptor has elapsed, a radiolabeled activator with a high affinity for the designer receptor is introduced into the organ or the region, which is subjected to PET imaging.

In the DREADD targeting of a G-protein-coupled muscarinic receptor, a mutated muscarinic receptor of which core receptor domain is genetically engineered has been used. Specifically, a specific region is infected with a viral vector into which a gene for a designer receptor is incorporated.

For example, a mutated human M4 muscarinic receptor (may be abbreviated as hM4D) or a mutated human M3 muscarinic receptor (may be abbreviated as hM3D) is introduced into a specific region in the brain of a live monkey subject. Expression of these muated muscarinic reseptor genes are confirmed by PET imaging using ¹¹C-labeled clozapine (hereinafter may be abbreviated as [¹¹C]clozapine) and ¹¹C-labeled clozapine-N-oxide (hereinafter may be abbreviated as [¹¹C]CNO), which are radiolabelled DREADD activators infused from an extracerebral blood vessel.

However, in conventional methods utilizing the [¹¹C]clozapine have some liminations of poor selectivity (specificity), because the [¹¹C]clozapine highly migrates into the brain through the blood-brain-barrier (BBB) but also acts on endogenous receptors in the brain. Furthermore, the act on endogenous receptors may cause side effects.

Meanwhile, the [¹¹C]CNO is less likely to act on the endogenous receptors than clozapine. However, the recent research revealed that the selectivity in the brain is unsatisfactory since the [¹¹C]CNO is metabolized in live animal subjects into the [¹¹C]clozapine and that a high metabolic degradability of the [¹¹C]CNO causes less detectable signals and unsatisfactory quantitativity (Non-Patent Documents 1 to 4) .

The present invention has been made in view of the above circumstances, and an object of the present invention is to provide a technique for imaging the brain of a live animal subject and applications thereof.

### Means for Solving the Problems

The present inventors have conducted intensive studies, and, as a result, have found a dibenzoazepine derivative having a high brain parmeability, a high receptor selectivity, and a high quantitativity. Thus, the present invention has been completed.

That is, the present invention is as follows.
(1-0) A compound represented by Formula (I) or a pharmaceutically acceptable salt or solvate thereof: 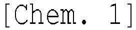 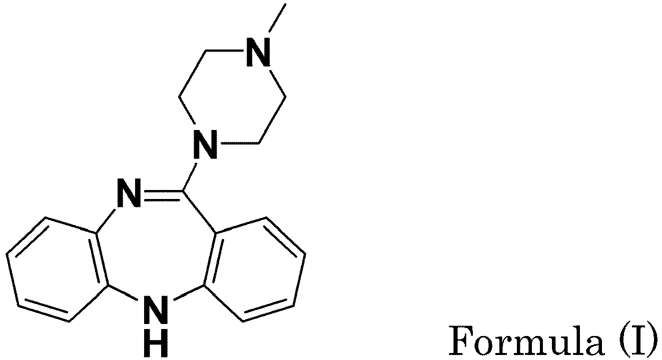 in which one or more atoms are or are not radioisotopes of the atom or atoms.
(1) A compound represented by Formula (I) or a pharmaceutically acceptable salt or solvate thereof: 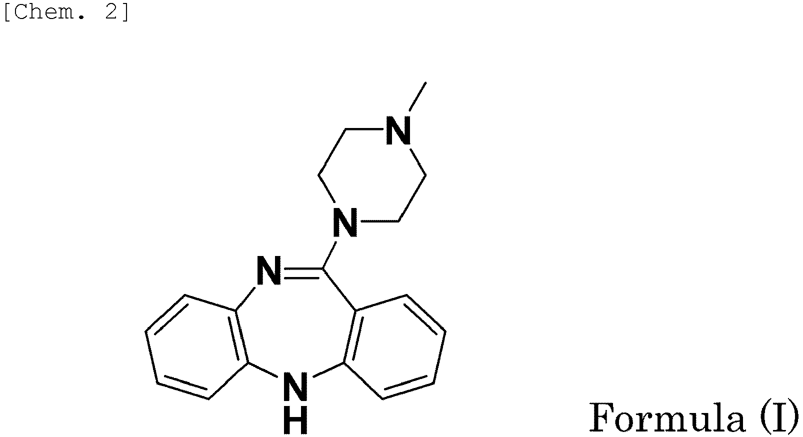 in which one or more atoms are radioisotopes of the atom or atoms.
(2) The compound according to (1) or a pharmaceutically acceptable salt or solvate thereof, in which the compound is a compound represented by Formula (II): 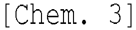 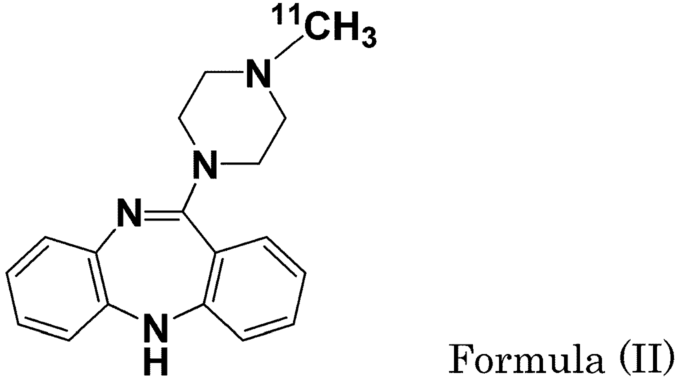
(3) A composition comprising:
   the compound or a pharmaceutically acceptable salt or solvate thereof according to (1) or (2).
   (3-1) A use of the compound or a pharmaceutically acceptable salt or solvate thereof according to (1) or (2), or the composition according to (3) in imaging of a mutated human M4 muscarinic acetylcholine receptor (hM4D) or a mutated human M3 muscarinic acetylcholine receptor (hM3D),
      the hM4D or the hM3D being an hM4D or an hM3D produced by expressing a gene encoding the mutated human M4 muscarinic acetylcholine receptor (hM4D) or the mutated human M3 muscarinic acetylcholine receptor (hM3D) introduced into a cell in a brain of a live animal subject.
   (3-2) A use of the compound or a pharmaceutically acceptable salt or solvate thereof according to (1) or (2), or the composition according to (3) in imaging of a mutated human M4 muscarinic acetylcholine receptor (hM4D) or a mutated human M3 muscarinic acetylcholine receptor (hM3D),
      the hM4D or the hM3D being an hM4D or an hM3D produced by expressing a gene encoding the mutated human M4 muscarinic acetylcholine receptor (hM4D) or the mutated human M3 muscarinic acetylcholine receptor (hM3D) introduced into a cell in a brain of a live animal subject, and
      the compound or a pharmaceutically acceptable salt or solvate thereof emitting radiation in a dose of 2 times or more for an hM4D-expressed site or 1.4 times or more for an hM3D-expressed site that of an unexpressed site as detected by imaging over a predetermined period from peripheral administration.
   (3-2-1) The use according to (3-2), in which the live animal subject is a live primate subject,
      the predetermined period is 30 to 90 minutes, and the dose to be detected is 6.3 g/cc or more for the hM4D-expressed site or 3.7 g/cc or more for the hM3D-expressed site when the dose is 2.5 g/cc or less for a whole brain including the unexpressed site as expressed as an index normalized to an amount of administration and a body weight.
   (3-3) A method for imaging an hM4D or an hM3D in a brain of a live animal subject, the method comprising: acquiring data on a distribution and/or an amount of expression of the hM4D or the hM3D in the brain containing a cell having an introduced gene encoding a mutated human M4 muscarinic acetylcholine receptor (hM4D) or a mutated human M3 muscarinic acetylcholine receptor (hM3D)
      in the live animal subject administered with the compound or a pharmaceutically acceptable salt or solvate thereof according to (1) or (2) that is allowed to migrate into the brain to selectively bind to the hM4D or the hM3D expressed by the gene, wherein
      the data is aquired by detecting radiation emitted from the compound or a pharmaceutically acceptable salt or solvate thereof selectively binding to the hM4D or the hM3D in the brain.
   (3-4) A method for imaging a brain activity of a live animal subject, the method comprising:
      acquiring data on modulation of an activity of an hM4D- or hM3D-expressing cell in a brain of the live animal subject containing a cell having an introduced gene encoding a mutated human M4 muscarinic acetylcholine receptor (hM4D) or a mutated human M3 muscarinic acetylcholine receptor (hM3D) in the live animal subject administered with the compound or a pharmaceutically acceptable salt or solvate thereof according to (1) or (2) that is allowed to migrate into the brain to selectively bind to the hM4D or the hM3D expressed by the gene.
   (3-5) An antagonist or an agonist comprising:
      a substance that selectively binds to a mutated human M4 muscarinic acetylcholine receptor (hM4D) or a mutated human M3 muscarinic acetylcholine receptor (hM3D) introduced into a cell in a brain of a live animal subject,
      the substance being the compound or a pharmaceutically acceptable salt or solvate thereof according to (1) or (2).
   (3-6) The agonist according to (3-5), in which the mutated receptor is the mutated human M3 muscarinic acetylcholine receptor (hM3D).
   (3-7) A pharmaceutical comprising:
      a substance that selectively binds to a mutated human M4 muscarinic acetylcholine receptor (hM4D) or a mutated human M3 muscarinic acetylcholine receptor (hM3D) introduced into a cell in a brain of a live primate subject,
      the substance being the compound or a pharmaceutically acceptable salt or solvate thereof according to (1) or (2).
   (3-8) A companion diagnostic agent comprising:
      a substance that selectively binds to a mutated human M4 muscarinic acetylcholine receptor (hM4D) or a mutated human M3 muscarinic acetylcholine receptor (hM3D) introduced into a cell in a brain of a live primate subject,
      the substance being the compound or a pharmaceutically acceptable salt or solvate thereof according to (1) or (2).
   (3-9) A method for imaging a nerve cell across a plurality of regions in a brain of a live animal subject,
      the nerve cell including a dendrite-containing body belonging to a first region and having axon terminals belonging to a region different from the first region,
      the first region having an introduced gene encoding a mutated human M4 muscarinic acetylcholine receptor (hM4D) or a mutated human M3 muscarinic acetylcholine receptor (hM3D), the method comprising:
      acquiring data on a distribution and/or an amount of expression of the hM4D or the hM3D in the axon terminal in the live animal subject administered with a radiolabeled compound or a pharmaceutically acceptable salt or solvate thereof according to (1) or (2) that is allowed to migrate into the brain to selectively bind to the hM4D or the hM3D, wherein the data is acquired by detecting radiation emitted from the radiolabeled compound or a pharmaceutically acceptable salt or solvate thereof according to (1) or (2) selectively binding to the hM4D or the hM3D.
   (3-10) A composition for imaging axon terminals of a nerve cell, into which a gene encoding a mutated human M4 muscarinic acetylcholine receptor (hM4D) or a mutated human M3 muscarinic acetylcholine receptor (hM3D) is introduced, in a brain of a live animal subject, the composition comprising: a radiolabeled compound or a pharmaceutically acceptable salt or solvate thereof according to (1) or (2).
(4) A composition for imaging a mutated human M4 muscarinic acetylcholine receptor (hM4D) or a mutated human M3 muscarinic acetylcholine receptor (hM3D) produced by expressing a gene encoding the hM4D or the hM3D introduced into a cell in a brain of a live animal subject, the composition comprising:
   a compound represented by Formula (III) or a pharmaceutically acceptable salt or solvate thereof: 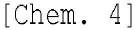 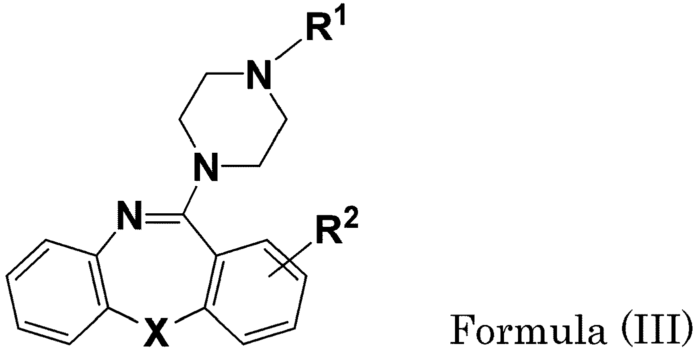
   in which R¹ represents hydrogen, alkyl, allyl, hydroxyalkyl, alkoxyalkyl, or alkoyloxyalkyl;
   the alkyl having 1 to 6 carbon atoms, the hydroxyalkyl having 1 to 2 carbon atoms, and the alkoxyalkyl and the alkoyloxyalkyl having 1 to 5 carbon atoms;
   X represents sulfur, sulfinyl, imino, methylene, or alkylimino;
   the alkylimino having 1 to 6 carbon atoms;
   R² represents hydrogen, halogen, hydroxy, trifluoromethyl, alkyl, alkoxy, alkylthio, nitro, amino, or aminosulfonyl;
   the alkyl, the alkoxy, and the alkylthio having 1 to 5 carbon atoms; and
   one or more atoms are radioisotopes of the atom or atoms.
(5) The composition according to (4), in which R¹ represents alkyl.
(6) The composition according to (5), in which X represents imino and R² represents hydrogen.
(7) A composition for imaging a mutated human M4 muscarinic acetylcholine receptor (hM4D) or a mutated human M3 muscarinic acetylcholine receptor (hM3D) produced by expressing a gene encoding the hM4D or the hM3D introduced into a cell in a brain of a live animal subject, the composition comprising:
   a dibenzodiazepine derivative or a pharmaceutically acceptable salt or solvate thereof,
   the dibenzodiazepine derivative is radiolabeled, and
   the derivative emitting radiation in a dose of 2 times or more for an hM4D-expressed site or 1.4 times or more for an hM3D-expressed site that of an unexpressed site as detected by imaging over a predetermined period from peripheral administration.
(8) The composition according to (7), in which the live animal subject is a live primate subject,
   the predetermined period is 30 to 90 minutes, and
   the dose to be detected is 6.3 g/cc or more for the hM4D-expressed site or 3.7 g/cc or more for the hM3D-expressed site when the dose is 2.5 g/cc or less for a whole brain including the unexpressed site as expressed as an index normalized to an amount of administration and a body weight.
(9-0) A method for imaging an hM4D or an hM3D in a brain of a live animal subject, the brain of the live animal containing a cell having an introduced gene encoding a mutated human M4 muscarinic acetylcholine receptor (hM4D) or a mutated human M3 muscarinic acetylcholine receptor (hM3D), the method comprising:
   allowing a compound or a pharmaceutically acceptable salt or solvate thereof to migrate into the brain to selectively bind to the hM4D or the hM3D when the compound or a pharmaceutically acceptable salt or solvate thereof is administered to the live animal subject, wherein the compound represented by Formula (III): 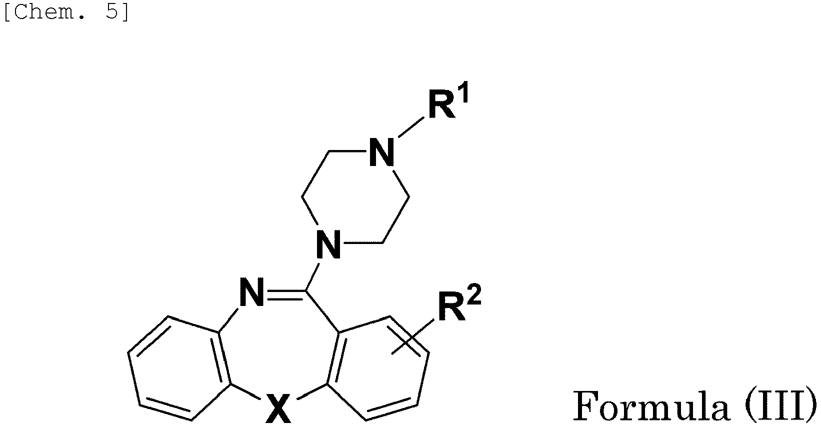 in which
   R¹ represents hydrogen, alkyl, allyl, hydroxyalkyl, alkoxyalkyl, or alkoyloxyalkyl;
   the alkyl having 1 to 6 carbon atoms, the hydroxyalkyl having 1 to 2 carbon atoms, and the alkoxyalkyl and the alkoyloxyalkyl having 1 to 5 carbon atoms;
   X represents sulfur, sulfinyl, imino, methylene, or alkylimino;
   the alkylimino having 1 to 6 carbon atoms;
   R² represents hydrogen, halogen, hydroxy, trifluoromethyl, alkyl, alkoxy, alkylthio, nitro, amino, or aminosulfonyl; the alkyl, the alkoxy, and the alkylthio having 1 to 5 carbon atoms; and one or more atoms are radioisotopes of the atom or atoms.
(9) A method for imaging an hM4D or an hM3D in a brain of a live animal subject, the method comprising aquiring data on a distribution and/or an amount of expression of the hM4D or the hM3D in the brain of the live animal subject containing a cell having an introduced gene encoding a mutated human M4 muscarinic acetylcholine receptor (hM4D) or a mutated human M3 muscarinic acetylcholine receptor (hM3D)
   in the live animal subject administered with a compound or a pharmaceutically acceptable salt or solvate thereof that selectively binds to the hM4D or the hM3D,
   the compound is represented by Formula (III):, 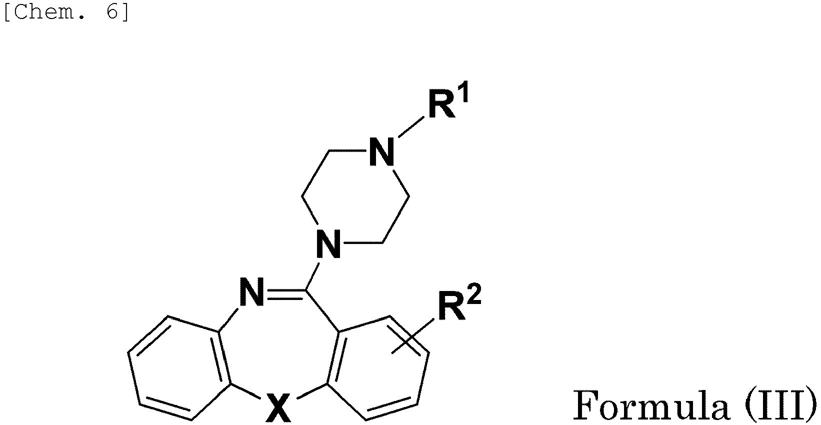 in which
   R¹ represents hydrogen, alkyl, allyl, hydroxyalkyl, alkoxyalkyl, or alkoyloxyalkyl;
   the alkyl having 1 to 6 carbon atoms, the hydroxyalkyl having 1 to 2 carbon atoms, and the alkoxyalkyl and the alkoyloxyalkyl having 1 to 5 carbon atoms;
   X represents sulfur, sulfinyl, imino, methylene, or alkylimino;
   the alkylimino having 1 to 6 carbon atoms;
   R² represents hydrogen, halogen, hydroxy, trifluoromethyl, alkyl, alkoxy, alkylthio, nitro, amino, or aminosulfonyl; the alkyl, the alkoxy, and the alkylthio having 1 to 5 carbon atoms; and
   one or more atoms are radioisotopes of the atom or atoms, wherein
   the compound or a pharmaceutically acceptable salt or solvate thereof is allowed to migrate into the brain to selectively bind to the hM4D or the hM3D expressed by the gene,and the data is acquired by detecting radiation emitted from the above-mentioned substance (the compound or a pharmaceutically acceptable salt or solvate thereof) selectively binding to the hM4D or the hM3D in the brain.
(9-1) A method for imaging according to (9-0), the method comprising:
   acquiring data on a distribution and/or an amount of expression of hM4D or hM3D in a brain by detecting radiation emitted from the compound selectively binding to the hM4D or the hM3D in the brain.
(9-2) A method for imaging an hM4D or an hM3D in a brain of a live animal subject, the method comprising: aquiring data on a distribution or an amount of expression of the hM4D or hM3D in the brain containing a cell having an introduced gene encoding a mutated human M4 muscarinic acetylcholine receptor (hM4D) or a mutated human M3 muscarinic acetylcholine receptor (hM3D)
   in the live animal subject administered with the composition according to (4) that is allowed to migrate into the brain to selectively bind to the hM4D or the hM3D, wherein the data is aquired by detecting radiation emitted from the compound or a pharmaceutically acceptable salt or solvate thereof selectively binding to the hM4D or the hM3D in the brain.
(10) A method for imaging a brain activity of a live animal subject, the method comprising:
   acquiring data on modulation of an activity of an hM4D- or hM3D-expressing cell in the brain containing a cell having an introduced gene encoding a mutated human M4 muscarinic acetylcholine receptor (hM4D) or a mutated human M3 muscarinic acetylcholine receptor (hM3D)
   in the live animal subject administered with a compound or a pharmaceutically acceptable salt or solvate thereof that selectively binds to the hM4D or the hM3D to the live animal subject, the compound being represented by Formula (III): 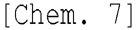 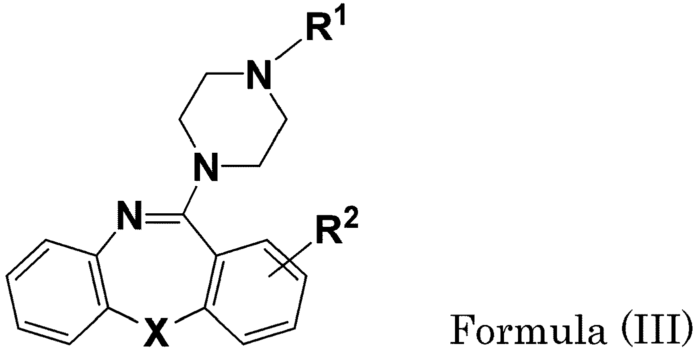
   in which R¹ represents hydrogen, alkyl, allyl, hydroxyalkyl, alkoxyalkyl, or alkoyloxyalkyl;
   the alkyl having 1 to 6 carbon atoms, the hydroxyalkyl having 1 to 2 carbon atoms, and the alkoxyalkyl and the alkoyloxyalkyl having 1 to 5 carbon atoms;
   X represents sulfur, sulfinyl, imino, methylene, or alkylimino;
   the alkylimino having 1 to 6 carbon atoms;
   R² represents hydrogen, halogen, hydroxy, trifluoromethyl, alkyl, alkoxy, alkylthio, nitro, amino, or aminosulfonyl; the alkyl, the alkoxy, and the alkylthio having 1 to 5 carbon atoms, wherein
   the compound or a pharmaceutically acceptable salt or solvate thereof is allowed to migrate into the brain to selectively bind to the hM4D or the hM3D expressed by the gene.
(10-1) The method for imaging according to (10), in which one or more atoms in the compound represented by Formula (III) are or are not radioisotopes of the atom or atoms.
(10-2) A method for imaging a brain activity of a live animal subject, the method comprising:
   acquiring data on modulation of an activity of an hM4D- or hM3D-expressing cell in the brain containing a cell having an introduced gene encoding a mutated human M4 muscarinic acetylcholine receptor (hM4D) or a mutated human M3 muscarinic acetylcholine receptor (hM3D)
   in the live animal subject administered with the composition according to (4) that is allowed to migrate into the brain to selectively bind to the hM4D or the hM3D expressed by the gene.
(10-3) The method for imaging according to (9-0), comprising acquiring data on modulation of an activity of an hM4D- or hM3D-expressing cell in the brain to image brain activity of a live animal subject using the compound or a pharmaceutically acceptable salt or solvate thereof that is allowed to migrate into the brain to selectively bind to the hM4D or the hM3D expressed by the gene.
(11) An antagonist or an agonist comprising:
   a substance that selectively binds to a mutated human M4 muscarinic acetylcholine receptor (hM4D) or a mutated human M3 muscarinic acetylcholine receptor (hM3D) introduced into a cell in a brain of a live animal subject,
   the substance being a compound represented by Formula (III) or a pharmaceutically acceptable salt or solvate thereof: 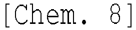 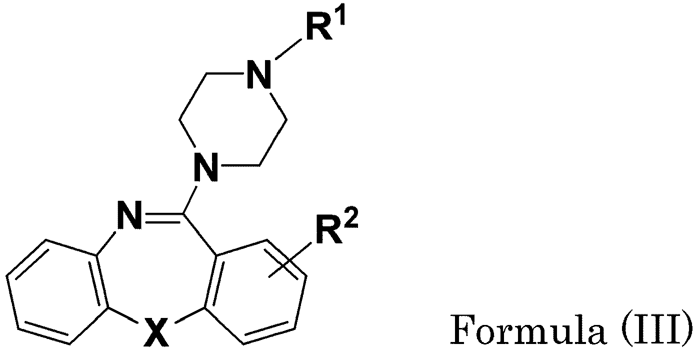
   in which R¹ represents hydrogen, alkyl, allyl, hydroxyalkyl, alkoxyalkyl, or alkoyloxyalkyl;
   the alkyl having 1 to 6 carbon atoms, the hydroxyalkyl having 1 to 2 carbon atoms, and the alkoxyalkyl and the alkoyloxyalkyl having 1 to 5 carbon atoms;
   X represents sulfur, sulfinyl, imino, methylene, or alkylimino;
   the alkylimino having 1 to 6 carbon atoms;
   R² represents hydrogen, halogen, hydroxy, trifluoromethyl, alkyl, alkoxy, alkylthio, nitro, amino, or aminosulfonyl;
   the alkyl, the alkoxy, and the alkylthio having 1 to 5 carbon atoms; and
   one or more atoms are or are not radioisotopes of the atom or atoms.
(12) The agonist according to (11), in which the mutated receptor is the mutated human M3 muscarinic acetylcholine receptor (hM3D).
(13) A pharmaceutical comprising:
   a substance that selectively binds to a mutated human M4 muscarinic acetylcholine receptor (hM4D) or a mutated human M3 muscarinic acetylcholine receptor (hM3D) introduced into a cell in a brain of a live primate subject,
   the substance being a compound represented by Formula (IV) or a pharmaceutically acceptable salt or solvate thereof: 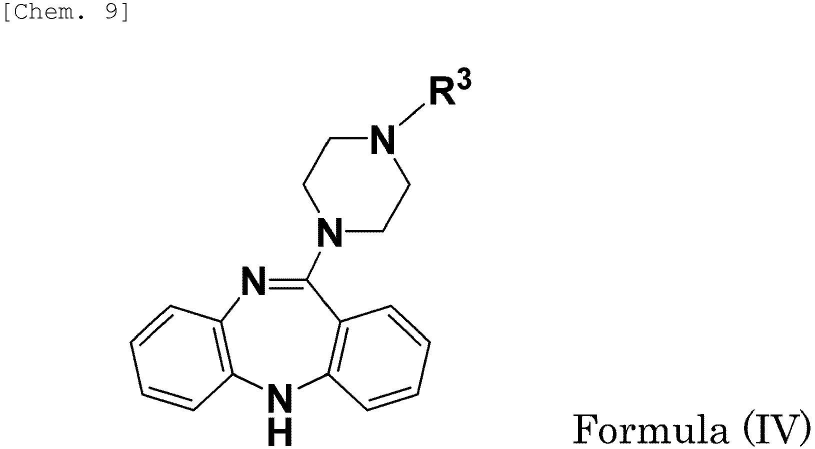 in which
   R³ represents hydrogen, alkyl, allyl, hydroxyalkyl, alkoxyalkyl, or alkoyloxyalkyl;
   the alkyl having 1 to 6 carbon atoms, the hydroxyalkyl having 1 to 2 carbon atoms, and the alkoxyalkyl and the alkoyloxyalkyl having 1 to 5 carbon atoms.
(14) A companion diagnostic agent comprising:
   a substance that selectively binds to a mutated human M4 muscarinic acetylcholine receptor (hM4D) or a mutated human M3 muscarinic acetylcholine receptor (hM3D) introduced into a cell in a brain of a live primate subject,
   the substance being a compound represented by Formula (IV) or a pharmaceutically acceptable salt or solvate thereof: 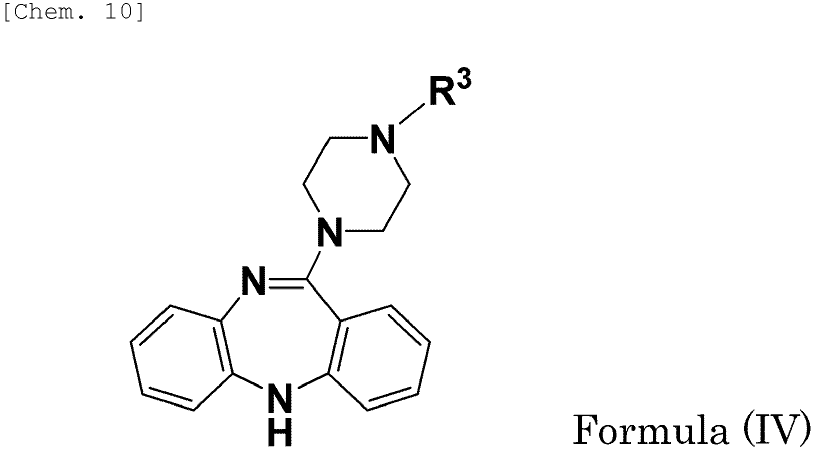
   in which R³ represents hydrogen, alkyl, allyl, hydroxyalkyl, alkoxyalkyl, or alkoyloxyalkyl;
   the alkyl having 1 to 6 carbon atoms, the hydroxyalkyl having 1 to 2 carbon atoms,
   and the alkoxyalkyl and the alkoyloxyalkyl having 1 to 5 carbon atoms;
   one or more atoms are or are not radioisotopes of the atom or atoms.
(15) A method for imaging a nerve cell across a plurality of regions in a brain of a live animal subject,
   the nerve cell including a dendrite-containing body belonging to a first region and having axon terminals belonging to a region different from the first region,
   the first region having an introduced gene encoding a mutated human M4 muscarinic acetylcholine receptor (hM4D) or a mutated human M3 muscarinic acetylcholine receptor (hM3D), the method comprising:
   acquiring data on a distribution and/or an amount of expression of the hM4D or the hM3D in the axon terminal in the live animal subject administered with a radiolabeled substance that is allowed to migrate into the brain to selectively bind to the hM4D or the hM3D, wherien
   the data is aquired by detecting radiation emitted from the substance selectively binding to the hM4D or the hM3D.
(16) A method for imaging a nerve cell across a plurality of regions in a brain of a live animal subject,
   the nerve cell including a dendrite-containing body belonging to a first region and having axon terminals belonging to a region different from the first region,
   the first region having an introduced gene encoding a mutated human M4 muscarinic acetylcholine receptor (hM4D) or a mutated human M3 muscarinic acetylcholine receptor (hM3D), the method comprising:
   acquiring data on a distribution and/or an amount of expression of the hM4D or the hM3D in the axon terminal in the live animal subject administered with a radiolabeled dibenzoazepine derivative that is allowed to migrate into the brain to selectively bind to the hM4D or the hM3D, wherein the data is aquired by detecting radiation emitted from the radiolabeled dibenzoazepine derivative selectively binding to the hM4D or the hM3D.
(16-1) The method for imaging according to (9), in which a nerve cell across a plurality of regions in the brain of the live animal subject includes a dendrite-containing body belonging to a first region and has axon terminals belonging to a region different from the first region,
   the first region has an introduced gene encoding a mutated human M4 muscarinic acetylcholine receptor (hM4D) or a mutated human M3 muscarinic acetylcholine receptor (hM3D), and the radiolabeled dibenzoazepine derivative is a compound represented by Formula (III) or a pharmaceutically acceptable salt or solvate thereof,
   the method comprising: detecting radiation emitted from the compound or a pharmaceutically acceptable salt or solvate thereof to acquire data on a distribution and/or an amount of expression of the hM4D or the hM3D in the axon terminals so that the nerve cell across a plurality of regions in the brain of the live animal subject are imaged.
(17) A composition for imaging axon terminals of a nerve cell, into which a gene encoding a mutated human M4 muscarinic acetylcholine receptor (hM4D) or a mutated human M3 muscarinic acetylcholine receptor (hM3D) is introduced, in a brain of a live animal subject, the composition comprisiing: a radiolabeled dibenzoazepine derivative or a pharmaceutically acceptable salt or solvate thereof.
(18) The composition according to (17) or the method for imaging according to (16), in which the dibenzoazepine derivative is a dibenzodiazepine derivative.
(19) The composition according to any of (7), (8), or (18), or the method for imaging according to (16) or (18), in which the dibenzodiazepine derivative is the compound represented by Formula (III).
(20) The composition according to any of (4) to (6) or (19), or the method for imaging according to (9), (10), or (19), or the antagonist or the agonist according to (11) or (12), in which the compound is the compound represented by Formula (IV) .
(21) The composition according to any of (4) to (6), (19), or (20), or the method for imaging according to (9), (10), (19), or (20), or the antagonist or the agonist according to (11), (12), or (20), the pharmaceutical according to (13), or the companion diagnostic agent according to (14), in which the compound is the compound represented by Formula (I) according to (1-0).
(22) The pharmaceutical according to (13), in which the compound is the compound represented by Formula (I) according to (1-0), exclusive of a radioisotope thereof.
(23) The composition according to any of (4) to (6), (19), (20), or (21), or the method for imaging according to (9), (10), (19), (20), or (21), or the antagonist or the agonist according to (11), (12), (20), or (21), or the companion diagnostic agent according to (14) or (21), in which the compound is the compound represented by Formula (I) according to (1).
(24) The composition according to any of (4) to (6), (19), (20), (21), or (23), or the method for imaging according to (9), (10), (19), (20), (21), or (23), or the antagonist or the agonist according to (11), (12), (20), (21), or (23), or the companion diagnostic agent according to (14), (21), or (23), in which the compound is the compound represented by Formula (II) according to (2).
(25) The composition, the method for imaging, the antagonist or the agonist, the pharmaceutical, or the companion diagnostic agent according to (4) to (20), in which the dibenzoazepine derivative, the dibenzodiazepine derivative compound, or the compound represented by Formula (III) or (IV) does not comprise the compound according to (1-0), (1), or (2) .

### Effects of the Invention

The present invention can provide a technique for imaging an hM4D or an hM3D receptor in an organ of a live animal subject such as a brain of a live animal subject, and applications thereof.

### BRIEF DESCRIPTION OF THE DRAWINGS

Fig. 1 is a microscale drawing showing a method for imaging a projection neuron according to the present invention;
Fig. 2 is a mesoscale drawing showing a method for imaging projection neurons according to the present invention;
Fig. 3 is an image taken by PET imaging using [¹¹C]C22b (binding potential) of an hM4D receptor expressed in the putamen;
Fig. 4 is an image taken by PET imaging using [¹¹C]clozapine (SUV) of an hM4D receptor expressed in the putamen;
Fig. 5 is an image taken by PET imaging using [¹¹C]C22b (SUV) of an hM4D receptor expressed in the putamen;
Fig. 6 is an image taken by PET imaging using [¹¹C]C22b (binding potential) of an hM3D receptor expressed in the left amygdala;
Fig. 7 is an image taken by PET imaging of glucose metabolism using [¹⁸F]FDG and C22b allowed to bind to an hM3D receptor expressed in the left amygdala;
Fig. 8 is an image in which an image (t-value) obtained by one-way repeated measures ANOVA for PET of glucose metabolism using [¹⁸F]FDG and C22b allowed to bind to an hM3D receptor expressed in the left amygdala is overlaid on an image of the brain structure;
Fig. 9 is an image taken by PET imaging using [¹¹C]C22b (SUV) in a projection target of a nerve cell expressing an hM4D receptor; and
Fig. 10 is an image taken by PET imaging using a compound represented by Chemical Formula (V) ([¹¹C]) (SUV) in a projection target of a nerve cell expressing an hM4D receptor.

### PREFERRED MODE FOR CARRYING OUT THE INVENTION

Embodiments of the present invention will now be described in detail.

### (1. Definitions)

The term "alkyl" in R¹ means a monovalent group that is produced when a saturated aliphatic hydrocarbon misses one hydrogen atom. The alkyl has 1 to 6 (C1-C6) carbon atoms, and typically has 1 to 5 (C1-C5), 1 to 4 (C1-C4), 1 to 3 (C1-C3), 1 to 2 (C1-C2), or 2 to 6 (C2-C6) carbon atoms. The alkyl may be linear or branched. Examples of the alkyl include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, t-butyl, pentyl, isopentyl, neopentyl, and hexyl. The alkyl may be further substituted with an appropriate substituent. Note that a hydrogen atom herein may be referred to as "hydrogen".

The term "allyl" in R¹ means -CH₂CH=CH₂.

The term "hydroxyalkyl" in R¹ means a group in which a part or all of hydrogen atoms in an alkyl group are substituted with hydroxy groups. The number of the hydroxy groups in the hydroxyalkyl is preferably 1 to 5 and most preferably 1.

The term "alkoxyalkyl" in R¹ means alkyl substituted with an alkoxy group having 1 to 3 (C1-C3) carbon atoms. The alkyl may be further substituted with an appropriate substituent. The alkyl means a monovalent group that is produced when a saturated aliphatic hydrocarbon misses one hydrogen atom and has 1 to 2 (C1-C2) carbon atoms and typically 1 to 2 (C1-C2) carbon atoms. The alkyl may be linear or branched. Examples of the alkoxyalkyl include, but are not limited to, methoxymethyl, methoxyethyl, and methoxypropyl.

The term "alkoyloxyalkyl" in R¹ means alkyl substituted with an alkoyloxy group (alkoxycarbonyl group) having 1 to 2 (C1-C2) carbon atoms. The term "alkoyl" may be referred to as alkanoyl. Alkyl substituted with an alkoxycarbonyl group instead of the alkoyloxy group may also be used. The alkyl may be further substituted with an appropriate substituent. The alkyl means a monovalent group that is produced when a saturated aliphatic hydrocarbon misses one hydrogen atom and has 1 to 3 (C1-C3) carbon atoms and typically 1 to 2 (C1-C2) carbon atoms. The alkyl may be linear or branched. Examples of the alkoyloxy include, but are not limited to, methanoyloxy and ethanoyloxy. Examples of the alkoxycarbonyl include, but are not limited to, methoxycarbonyl and ethoxycarbonyl.

The term "sulfinyl" in X means -(S=O)-.

The term "imino" in X means -(NH)-.

The term "methylene" in X means -(CH₂)-.

The term "alkylimino" in X means -(NR⁴)-. R⁴ is alkyl, which may be further substituted with an appropriate substituent. The alkyl means a monovalent group that is produced when a saturated aliphatic hydrocarbon misses one hydrogen atom and has 1 to 3 (C1-C3) carbon atoms and typically 1 to 2 (C1-C2) carbon atoms. The alkyl may be linear or branched. Examples of the alkyl include, but are not limited to, methyl, ethyl, propyl, and isopropyl.

The term "halogen" in R² means fluoro (-F), chloro (-Cl), bromo (-Br), and iodo (-I). Note that the term "halogen", as used herein, includes a halogen atom.

The term "alkyl" in R² means a monovalent group that is produced when a saturated aliphatic hydrocarbon misses one hydrogen atom. The alkyl has 1 to 5 (C1-C5) carbon atoms, and typically has 1 to 4 (C1-C4), 1 to 3 (C1-C3), 1 to 2 (C1-C2), or 2 to 5 (C2-C5) carbon atoms. The alkyl may be linear or branched. Examples of the alkyl include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, t-butyl, pentyl, isopentyl, and neopentyl. The alkyl may be further substituted with an appropriate substituent.

The term "alkoxy" in R² means - (O-R⁵) -. R⁵ is alkyl, which may be further substituted with an appropriate substituent. The alkyl has 1 to 5 (C1-C5) carbon atoms, and typically has 1 to 4 (C1-C4), 1 to 3 (C1-C3), 1 to 2 (C1-C2), or 2 to 5 (C2-C5) carbon atoms. The alkyl may be linear or branched. Examples of the alkoxy include, but are not limited to, methoxy, ethoxy, and propanoxy.

The term "alkylthio" in R² means -SR⁶. R⁶ is alkyl, which may be further substituted with an appropriate substituent. The alkyl has 1 to 5 (C1-C5) carbon atoms, and typically has 1 to 4 (C1-C4), 1 to 3 (C1-C3), 1 to 2 (C1-C2), or 2 to 5 (C2-C5) carbon atoms. The alkyl may be linear or branched. Examples of the alkylthio include, but are not limited to, methylthio, methoxy, ethylthio, and propanethio.

The term "nitro" in R² means -NO₂.

The term "amino" in R² means any of -NH₂, -NHR⁷, and - NR⁷R⁸. Here, R⁷ and R⁸ are alkyls, R⁷ and R⁸ may be the same as or different from each other, and may be further substituted with an appropriate substituent. The alkyl has 1 to 5 (C1-C5) carbon atoms, and typically has 1 to 4 (C1-C4), 1 to 3 (C1-C3), 1 to 2 (C1-C2), or 2 to 5 (C2-C5) carbon atoms. The alkyl may be linear or branched. Examples of the amino include, but are not limited to, an amino group (-NH₂). The -NHR⁷ and the - NR⁷R⁸ herein may be collectively referred to as the term "alkylamino".

The term "aminosulfonyl" in R² means -SO₂NR⁹R¹⁰. Here, R⁹ and R¹⁰ are selected from hydrogen, methyl, and -SONR¹¹, and may be the same as or different from each other. Here, R¹¹ is C1-C2 alkyl or -SO₂NR¹² and R¹² is C1-C2 alkyl. Examples of the aminosulfonyl include, but are not limited to, -SO₂N(CH₃)₂ and -SO₂NHCH₃.

The term "pharmaceutically acceptable salt" indicates a salt that is not harmful to live animal subjects, in particular, mammals. The pharmaceutically acceptable salt can be formed with a non-toxic acid or a base including an inorganic acid or an inorganic base, or an organic acid or an organic base. The pharmaceutically acceptable salt includes an acid addition salt and a base addition salt.

Examples of the acidic salt include salts with alkali metals such as sodium, potassium, and lithium; salts with alkali earth metals such as calcium and magnesium; salts with metals such as aluminum and zinc; ammonium salts; and salts with nitrogen-containing organic bases such as trimethylamine, triethylamine, tributylamine, pyridine, N,N-dimethylaniline, N-methylpiperidine, N-methylmorpholine, diethylamine, diethanolamine, ethylenediamine, dicyclohexylamine, procaine, chloroprocaine, dibenzylamine, N-benzyl-β-phenethylamine, 1-ephenamine and N,N'-dibenzylethylenediamine, meglumine (N-methylglucamine).

Examples of the basic salt include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalene sulfonic acid.

The term "solvate" means a solvent-containing compound that is formed by association of one or a plurality of solvent molecules with the compound of the present invention. The solvate include, for example, a monosolvate, a disolvate, a trisolvate, and a tetrasolvate. Furthermore, the solvate includes a hydrate. The solvate may include at least one selected from the group consisting of a monosolvate, a disolvate, a trisolvate, and a tetrasolvate; and preferably is at least one selected from the group consisting of a monosolvate, a disolvate, a trisolvate, and a tetrasolvate.

For the term "a compound or a pharmaceutically acceptable salt thereof", when the compound has an isomer (for example, enantiomer, geometric isomer, and tautomer), the present invention includes all the isomers. Furthermore, the present invention may include hydrates, solvates, and all crystalline forms.

The term "agent" includes a pharmaceutical, food for medical use, food, and a dietary supplement. The term "food" also includes functional food, health food, and healthful food.

The term "animal", as used herein, includes a mammal or a non-mammal. Furthermore, the mammal includes a rodent and a non-rodent. The non-rodent includes a primate. For example, the rodent is rat, mouse, guinea pig, or rabbit. Furthermore, those which are the non-rodents but not the primates are, for example, dog, cat, or miniature swine. The animal may be categorized into human and non-human.

The term "primate", as used herein, is, for example, human or a monkey (marmoset (e.g., common marmoset), rhesus macaque, and cynomolgus). The primate may be categorized into human and non-human.

The term "brain", as used herein, has a meaning commonly used by one skilled in the art, and, for example, indicates the cerebrum, the diencephalon, the cerebellum, or the mesencephalon. More specifically, the term means the cerebral cortex, for example, the limbic cortex. Further specifically, the term means the cingulate gyrus, the amygdala, the hippocampus, the septum, the fornix, the mammillary body, or the parahippocampal gyrus.

The terms "human M4 muscarinic acetylcholine receptor" and "human M3 muscarinic acetylcholine receptor" are known to one skilled in the art and herein mean subtypes M4 and M3 of human-derived metabotropic receptors, respectively. The "human M4 muscarinic acetylcholine receptor" and "human M3 muscarinic acetylcholine receptor" may be abbreviated as "hM4" and "hM3", respectively.

The term "mutated human M4 muscarinic receptor" and "mutated human M3 muscarinic receptor" are also known to one skilled in the art and herein may be abbreviated as "hM4D" and "hM3D", respectively. Here, the mutated human M4 muscarinic receptor or the mutated human M3 muscarinic receptor is produced by introducing a viral vector including a gene encoding an hM4D or an hM3D into a desired site of a mammal and expressing the hM4D or the hM3D. In the present invention, the gene encoding an hM4D and the gene encoding an hM3D may be used separately or simultaneously.

The mutated human M4 muscarinic acetylcholine receptor (hM4D) and the gene encoding an hM4D are known to one skilled in the art. As described in Sequence Listings, the hM4D has an amino acid sequence (SEQ ID NO:2) in which tyrosine (Tyr) at position 113 is mutated into cysteine (Cys) and alanine (Ala) at position 203 is mutated into glycine (Gly) in an amino acid sequence of the human M4 muscarinic acetylcholine receptor (hM4) (SEQ ID NO:1). The gene encoding an hM4D has an nucleotidesequence of SEQ ID NO:3. The hM4D does not bind to an endogenous transmitter such as a neurotransmitter.

Furthermore, the mutated human M3 muscarinic acetylcholine receptor (hM3D) and the gene encoding an hM3D are also known to one skilled in the art. The hM3D has an amino acid sequence (SEQ ID NO:5) in which tyrosine (Tyr) at position 149 is mutated into cysteine (Cys) and alanine (Ala) at position 239 is mutated into glycine (Gly) in an amino acid sequence of the human M3 muscarinic acetylcholine receptor (hM3) (SEQ ID NO:4). The gene encoding an hM3D has an nucleotidesequence of SEQ ID NO:6. The hM3D does not bind to an endogenous transmitter such as a neurotransmitter.

The gene encoding an hM4D or an hM3D may be linked to a promoter, an enhancer, and/or Poly(A), etc., as necessary. Preferably, a Thy-1-promoter specifically expressed in a nerve cell may be linked. Preferably, the gene encoding an hM4D or an hM3D is linked to the Thy-1-promoter at the upstream side thereof and SV40 at the downstream side thereof.

The term "imaging", as used herein, means molecular imaging, and examples thereof include, but are not limited to, positron emission tomography (PET), a multi-photon imaging method, a two-photon imaging method, a near-infrared fluorescence imaging method, autoradiography, single photon emission computed tomography (SPECT), and the like. Among them, PET imaging is preferable.

### (2. Compound)

The present invention provides the compound represented by Formula (I), which is a dibenzodiazepine derivative, or a pharmaceutically acceptable salt or solvate thereof. Here, in the formula, one or more atoms are or are not radioisotopes of the atom or atoms. Specifically, when the compound represented by Formula (I) includes a radioisotope, a carbon atom may be ¹¹C and a nitrogen atom may be ¹³N. There may be one ¹¹C or one ¹³N. The radioisotope in the compound represented by Formula (I) is preferably a radioisotope of a carbon atom, more preferably ¹¹C, and, further preferably one ¹¹C. Furthermore, in one embodiment of the present invention, variation of the compound represented by Formula (I) includes the compound represented by Formula (II) or a pharmaceutically acceptable salt or solvate thereof. The compound represented by Formula (I) may not include a radioisotope.

### (3. Synthesis method)

The compound represented by Formula (II) (may be referred to as [¹¹C]C22b) can be produced by the following synthesis method (Formula (V)).

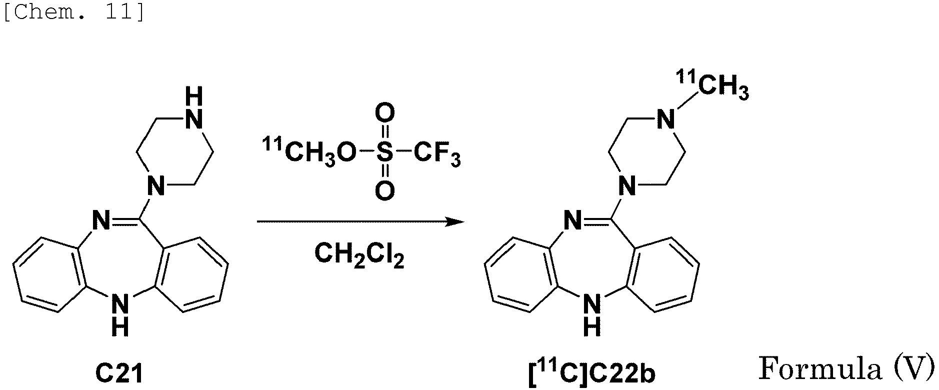

As starting materials, 0.2 mg of 11-(piperazine-1-yl)-5H-dibenzo[b,e][1,4]diazepine (may be referred to as C21) was dissolved in 0.3 mL of dichloromethane anhydride and charged into a reaction container for synthesizing a [¹¹C] label. To the resultant mixed solution, [¹¹C]methyltriflate radiolabeled with the normal method ([¹¹C]MeOTf) was collected into the reaction container, which had been cooled, under a nitrogen gas stream, followed by saturating radioactivity and allowed to react at room temperature for 5 minutes. After the reaction, the solvent was distilled off using a nitrogen gas stream and a residue was collected. Then, 0.5 mL of a solvent for HPLC separation (acetonitrile/water = 40/60, solvent containing 0.1% triethylamine) was added to the thus-collected residue and a RI peak portion of the target [¹¹C]C22b was fractionated using high performance liquid chromatography (HPLC). When used in animal experimentations, the thus-fractionated solution was concentrated and collected in saline containing a surfactant Tween80 (Polysorbate 80).

### (4. Composition)

The present invention provides a composition including the compound represented by Formula (I), or the compound represented by Formula (II), or a pharmaceutically acceptable salt or solvate thereof. The composition may be included in a pharmaceutically acceptable carrier. Examples of the pharmaceutically acceptable carrier include, but are not limited to, sterilized water, brine, saline or phosphate buffered saline (PBS), a sodium chloride injection solution, a Ringer's injection solution, an isotonic dextrose injection solution, a sterile water injection solution, dextrose, and a lactated Ringer's injection solution. The composition can be administered parenterally, intravenously, or intraperitoneally, but not particularly limited thereto. The above-mentioned substance ("compound or a pharmaceutically acceptable salt or solvate thereof") is transported through blood vessels and then allowed to migrate through the blood-brain barrier into the brain. Therefore, the substance needs to be capable of passing through the blood-brain barrier, so preferably has a predetermined low molecular weight, lipid solubility, as well as a predetermined blood solubility. Note that the substance may be a single substance or may be loaded on a drug delivery system (DDS). Note that an amount of administration of the substance may be appropriately determined based on a type of a substance to be used; the age, weight, health conditions, sex, and diet of a subject that receives administration; the number of administration; and a route of administration, etc. The administration of the substance is not particularly limited.

### (5. Composition for imaging hM4D or hM3D [1])

The present invention provides a composition including the compound represented by Formula (III) or a pharmaceutically acceptable salt or solvate thereof as a composition for imaging an M4D or an hM3D produced by introducing a gene encoding the hM4D or the hM3D into a cell in a brain of a live animal subject and expressing the gene. Here, in the formula, one or more atoms are radioisotopes of the atom or atoms. As one example of the radioisotopes, a carbon atom may be ¹¹C, a nitrogen atom may be ¹³N, an oxygen atom may be ¹⁵O, and a fluorine atom, which is one example of halogen, may be ¹⁸F. Here, the composition may be included in a pharmaceutically acceptable carrier, as mentioned in (4. Composition) above.

In the compound represented by Formula (III), R¹ is preferably C1-C6 alkyl, X is preferably sulfur, sulfinyl, imino, methylene, or C1-C6 alkylimino, and R² is preferably hydrogen, halogen, hydroxy, trifluoromethyl, C1-C5 alkyl, C1-C5 alkoxy, C1-C5 alkylthio, nitro, amino, or aminosulfonyl. In one embodiment of the present invention, as a combination of substituents in the compound represented by Formula (III), R¹ is C1-C6 alkyl,
X is sulfur, sulfinyl, imino, methylene, or C1-C6 alkylimino, R² is hydrogen, halogen, hydroxy, trifluoromethyl, C1-C5 alkyl, C1-C5 alkoxy, C1-C5 alkylthio, nitro, amino, or aminosulfonyl, and
one or more atoms are radioisotopes of the atom or atoms. As one example of the radioisotopes, a carbon atom may be ¹¹C, a nitrogen atom may be ¹³N, an oxygen atom may be ¹⁵O, and a fluorine atom, which is one example of halogen, may be ¹⁸F.

Furthermore, in another embodiment, as a combination of substituents in the compound represented by Formula (III), R¹ is C1-C6 alkyl,
X is imino,
R² is hydrogen, halogen, hydroxy, trifluoromethyl, C1-C5 alkyl, C1-C5 alkoxy, C1-C5 alkylthio, nitro, amino, or aminosulfonyl, and
one or more atoms are radioisotopes of the atom or atoms. As one example of the radioisotopes, a carbon atom may be ¹¹C, a nitrogen atom may be ¹³N, an oxygen atom may be ¹⁵O, and a fluorine atom, which is one example of halogen, may be ¹⁸F.

Furthermore, in another embodiment, as a combination of substituents in the compound represented by Formula (III), R¹ is C1-C6 alkyl,
X is sulfur, sulfinyl, imino, methylene, or C1-C6 alkylimino, R² is hydrogen, and
one or more atoms are radioisotopes of the atom or atoms. As one example of the radioisotopes, a carbon atom may be ¹¹C, a nitrogen atom may be ¹³N, and an oxygen atom may be ¹⁵O.

Furthermore, in another embodiment, as a combination of substituents in the compound represented by Formula (III), R¹ is C1-C6 alkyl,
X is imino,
R² is hydrogen, and
one or more atoms are radioisotopes of the atom or atoms. As one example of the radioisotopes, a carbon atom may be ¹¹C and a nitrogen atom may be ¹³N.

Furthermore, in another embodiment, as a combination of substituents in the compound represented by Formula (III), R¹ is methyl,
X is imino,
R² is hydrogen, and one or more atoms are radioisotopes of the atom or atoms. As one example of the radioisotopes, a carbon atom may be ¹¹C and a nitrogen atom may be ¹³N.

In one embodiment, a compound in which one or more atoms in 11-(piperazine-1-yl)-5H-dibenzo[b,e] [1,4]diazepine and 6-(4-methylpiperazine-1-yl)-11H-benzo[c] [1]benzazepine are radioisotopes of the atom or atoms may be excluded from the compound represented by (III).

Furthermore, in another embodiment, a combination of substituents in the compound represented by Formula (III) may be a composition including the compound represented by Formula (II) .

The above description for the compound represented by Formula (III) can be applied to compounds for use in (6. Composition for imaging hM4D or hM3D [2]), (7. Method for imaging hM4D or hM3D in brain), (8. Method for imaging change in brain activity associated with modulation of activity of hM4D- or hM3D-expressing cell in brain), (9. Agonist/Antagonist), (11. Method for imaging axon terminal of nerve cell across a plurality of regions), and (12. Composition for imaging axon terminal of nerve cell across a plurality of regions) described below. Furthermore, the above description for the compound represented by Formula (III) can be applied to compounds for use in (10. Pharmaceutical) described below, except that they do not include a radioisotope.

Compositions including these compounds can specifically bind to an hM4D or an hM3D produced by expressing a gene encoding the hM4D or the hM3D, which has been introduced into a cell in the brain of a live animal subject, in the cell.

Furthermore, the compositions including these compounds can pass through the blood-brain barrier (BBB) in the brain of the live animal subject. In other words, the compositions including these compounds can be administered from blood vessels downstream to the BBB, for example, peripheral blood vessels of hands and feet, preferably by intravenous injection. When the compositions are administered from the blood vessels, the compositions can specifically bind to the hM4D or the hM3D expressed and produced in the cell. As used herein and the claims, a downstream of the BBB may be referred to as periphery and administration from the downstream of the BBB may be referred to as peripheral administration.

Furthermore, the compositions including these compounds have a higher binding property for the hM4D or the hM3D, but have a lower binding property for endogenous substances such as wild-type receptors. Therefore, the compositions have high selectivity for the hM4D or the hM3D. When a binding potential of the compounds included in these compositions is converted to a numerical form, for example, when a vector for expressing an hM4D receptor or an hM3D receptor in a nerve cell-specific manner (e.g., adeno-associated virus (AAV) vector (AAV2-CMV-hM4D)) is injected and an image of a ligand binding potential is made using the cerebellum as a reference region (a binding potential (BP) in the cerebellum is determined as 1) from PET data taken for 90 minutes, a region into which the vector has been injected shows a significantly higher binding property than, that is, 3.4 to 1.1 times that of a control region (uninjected region) opposite to the region into which the vector has been injected. The binding potential may be, for example, about 11 times in the case of the hM4D receptors (the regions are left putamen/right putamen) and about 3.4 times in the case of the hM3D receptors (the regions are right amygdala/left amygdala). Such a binding potential can be applied to compounds for use in (6. Composition for imaging hM4D or hM3D [2]), (7. Method for imaging hM4D or hM3D in brain), (8. Method for imaging change in brain activity associated with modulation of activity of hM4D- or hM3D-expressing cell in brain), (9. Agonist/Antagonist), (11. Method for imaging axon terminal of nerve cell across a plurality of regions), and (12. Composition for imaging axon terminal of nerve cell across a plurality of regions) described below.

Furthermore, the compositions including these compounds may be easily excreted from an animal body. In other words, when the compositions including these compounds are administered from blood vessels downstream to the BBB, for example, peripheral blood vessels of hands and feet, preferably by intravenous injection, the hM4D or the hM3D expressed and produced in the cell can be stably imaged without toxicity. Furthermore, the compositions including these compounds emit radiation with high signal intensity. In other words, when the compositions including these compounds are administered from blood vessels downstream to the BBB, for example, peripheral blood vessels of hands and feet, preferably by intravenous injection, the hM4D or the hM3D expressed and produced in the cell can be quantitatively PET-imaged.

### (6. Composition for imaging hM4D or hM3D [2])

The present invention provides a composition including, as a composition for imaging an M4D or an hM3D produced by introducing a gene encoding the hM4D or the hM3D into a cell in a brain of alive animal subject and expressing the gene, a dibenzodiazepine derivative or a pharmaceutically acceptable salt or solvate thereof,
the dibenzodiazepine derivative being radiolabeled, and the derivative emitting radiation in a dose of 2 times or more for an hM4D-expressed site or 1.4 times or more for an hM3D-expressed site that of an unexpressed site as detected by imaging over a predetermined period from peripheral administration.

Here, the dibenzodiazepine derivative means a compound having a dibenzodiazepine skeleton. The composition may be included in a pharmaceutically acceptable carrier, as mentioned in (4. Composition). Furthermore, when the imaging is PET imaging, one or more atoms in the compound are radioisotopes of the atom or atoms. As one example of the radioisotopes, a carbon atom may be ¹¹C and a nitrogen atom may be ¹³N.

Examples of the dibenzodiazepine skeleton include dibenzodiazepine or a dibenzodiazepine derivative including or including, as a core, 1,2-diazepine, 1,3-diazepine, or 1,4-diazepine. In one embodiment of the present invention, the dibenzodiazepine skeleton is a skeleton including 1,4-diazepine. One example of the dibenzodiazepine derivative is dibenzo[b,e] [1,4]diazepine.

The dibenzodiazepine skeleton may be substituted with an appropriate substituent. Examples of the appropriate substituent that the dibenzodiazepine skeleton may have include alkyl, acyl, aryl, a nitrogen-containing heterocycle, a sulfur-containing heterocycle, an oxygen-containing heterocycle, and halogen. The nitrogen-containing heterocycle may be aromatic or non-aromatic. The non-aromatic nitrogen-containing heterocycle is, for example, a nitrogen-containing aliphatic hydrocarbon cycle, and preferably a nitrogen-containing saturated aliphatic hydrocarbon cycle. Furthermore, these substituents may be further substituted with appropriate substituents.

In one embodiment, a compound in which one or more atoms in 11-(piperazine-1-yl)-5H-dibenzo[b,e] [1,4]diazepine are radioisotopes of the atom or atoms may be excluded.

Compositions including these compounds can specifically bind to an hM4D or an hM3D produced by expressing a gene encoding the hM4D or the hM3D, which has been introduced into a cell in a predetermined organ of a live animal subject, in the cell. Furthermore, the compositions including these compounds can pass through the blood-brain barrier (BBB) in the brain of the live animal subject. In other words, the compositions including these compounds can be administered from blood vessels downstream to the BBB, for example, peripheral blood vessels of hands and feet, preferably by intravenous injection. When the compositions are administered from the blood vessels, the compositions specifically can bind to the hM4D or the hM3D, which is expressed and produced in the cell.

When the hM4D is only expressed in a cell in a part of, but not all of, regions of the organ (hereinafter may be referred to as site), the compositions including these compound are peripherally administered, and the predetermined organ is imaged, the compositions emit radiation in an hM4D-expressed site in a dose of 2 times or more that of an unexpressed site as detected by imaging over a predetermined period. In other words, the compositions exhibit selectivity for the hM4D-expressed site 2 times or more that of the unexpressed site in imaging of the organ of the live animal subject. The dose or the selectivity may be, for example, 5 times or less.

Alternatively, the hM3D is expressed only in a cell in a part of, but not all of, regions of the organ, the compositions including these compound are peripherally administered, and the predetermined organ is imaged, the compositions emit radiation in an hM3D-expressed site in a dose of 1.4 times or more that of an unexpressed site as detected by imaging over a predetermined period. In other words, the compositions exhibit selectivity for the hM3D-expressed site 1.4 times or more that of the unexpressed site in imaging of the organ of the live animal subject. The dose or the selectivity may be, for example, 5 times or less.

Note that the dose is calculated from an integrated value (integration value) of signals detected by imaging over a predetermined period. The selectivity may be determined by performing detection in both the unexpressed site and the expressed site and dividing a detected dose of the expressed site by that of the unexpressed site. Note that, when background noise is generated to the extent counted as a signal in the measurement, the background noise is subtracted from each detected dose, which is subjected to the calculation.

Thus, when using the compositions exhibiting the selectivity for the hM4D-expressed site 2 times or more or for the hM3D-expressed site 1.4 times or more that of the unexpressed site in the organ of the live animal subject, the hM4D-expressed site or the hM3D-expressed site can be satisfactorily imaged, and whether the hM4D or the hM3D is expressed or not can be easily determined. Furthermore, an amount of expression can also be quantitatively measured.

Moreover, there is a range within which quantification stability can be obtained. Specifically, the selectivity for the hM4D-expressed site is desirably 2.1 times or more, more desirably 2.2 times or more, further desirably 2.3 times or more, further more desirably 2.31 times or more, and, for example, 5 times or less that of the unexpressed site. Furthermore, the selectivity for the hM3D-expressed site is desirably 1.41 times or more, more desirably 1.43 times or more, further desirably 1.45 times or more, and, for example, 5 times or less that of the unexpressed site. The selectivity falling within the above numeral range improves a signal-to-noise intensity ratio (S/N ratio) of an image and allows stable quantitative evaluation. The term "stable" means measurement is universal under the same conditions, for example, repeated reproducibility.

Furthermore, when the live animal subject is a live primate subject and the organ is a brain, the imaging is performed for 30 to 90 minutes from peripheral administration of the compound, and radiation from the composition including a dibenzodiazepine derivative or a pharmaceutically acceptable salt or solvate thereof is detected in a dose of 6.3 g/cc or more for the hM4D-expressed site or 3.7 g/cc or more for the hM3D-expressed site when the dose is 2.5 g/cc or less for a whole brain including the unexpressed site as expressed as an index normalized to an amount of the administration and a body weight of the live subject that receives the administration.

The above description for the dose and selectivity can be applied to compounds for use in (5. Composition for imaging hM4D or hM3D [1]) described above, and (7. Method for imaging hM4D or hM3D in brain), (8. Method for imaging change in brain activity associated with modulation of activity of hM4D- or hM3D-expressing cell in brain), (9. Agonist/Antagonist), (11. Method for imaging axon terminal of nerve cell across a plurality of regions), and (12. Composition for imaging axon terminal of nerve cell across a plurality of regions) described below.

When the imaging is PET imaging, one embodiment of the present invention is the compound in which a dibenzodiazepine skeleton is substituted with a nitrogen-containing heterocycle. The nitrogen-containing heterocycle may be aromatic or non-aromatic. The non-aromatic nitrogen-containing heterocycle is, for example, a nitrogen-containing aliphatic hydrocarbon cycle, and preferably a nitrogen-containing saturated aliphatic hydrocarbon cycle. Furthermore, one or more atoms in the compound are radioisotopes of the atom or atoms. As one example of the radioisotopes, a carbon atom may be ¹¹C and a nitrogen atom may be ¹³N.

One example of the nitrogen-containing heterocycle is piperazine. For example, a dibenzodiazepine skeleton in 11-piperazino-5H-dibenzo[b,e][1,4]diazepine may be substituted with piperazine at position 11.

The nitrogen-containing heterocycle substituted for the dibenzodiazepine skeleton may be further substituted with an appropriate substituent. As one example, in the case of 11-piperazino-5H-dibenzo[b,e][1,4]diazepine, a nitrogen on the piperazine skeleton may be substituted with an alkyl group. One example of the alkyl group, which is a substituent in the above example, includes a C1-C6 alkyl group, specifically, a methyl group.

The compositions including these compounds may be easily excreted from an animal body. In other words, when the compositions including these compounds are administered from blood vessels downstream to the BBB, for example, peripheral blood vessels of hands and feet, preferably by intravenous injection, the hM4D or the hM3D expressed and produced in the cell can be stably imaged without toxicity. Furthermore, the compositions including these compounds emit radiation with high signal intensity. In other words, when the compositions including these compounds are administered from blood vessels downstream to the BBB, for example, peripheral blood vessels of hands and feet, preferably by intravenous injection, the hM4D or the hM3D expressed and produced in the cell can be quantitatively PET-imaged.

### (7. Method for imaging hM4D or hM3D in brain)

The present invention provides a method for imaging an hM4D or an hM3D in a brain of a live animal subject, the method comprising: introducing a gene encoding a mutated human M4 muscarinic acetylcholine receptor (hM4D) or a mutated human M3 muscarinic acetylcholine receptor (hM3D) into a cell in a brain of a live animal subject; administering the compound represented by Formula (III) that selectively binds to the hM4D or the hM3D or a pharmaceutically acceptable salt or solvate thereof to the live animal subject;
allowing the compound or a pharmaceutically acceptable salt or solvate thereof to migrate into the brain to thereby allow the compound or a pharmaceutically acceptable salt or solvate thereof to selectively bind to the hM4D or the hM3D expressed by the gene; and detecting radiation emitted from the above-mentioned substance (the compound or a pharmaceutically acceptable salt or solvate thereof) selectively binding to the hM4D or the hM3D in the brain to thereby acquire a datum on a distribution and/or an amount of expression of the hM4D or the hM3D in the brain.

Here, one or more atoms in the compound represented by Formula (III) are radioisotopes of the atom or atoms. As one example of the radioisotopes, a carbon atom may be ¹¹C, a nitrogen atom may be ¹³N, an oxygen atom may be ¹⁵O, and a fluorine atom, which is one example of halogen, may be ¹⁸F.

In one embodiment, a compound in which one or more atoms in 11-(piperazine-1-yl)-5H-dibenzo[b,e] [1,4]diazepine, and 6-(4-methylpiperazine-1-yl)-11H-benzo[c] [1]benzazepine are radioisotopes of the atom or atoms may be excluded.

Furthermore, the composition may be included in a pharmaceutically acceptable carrier, as mentioned in (4. Composition).

The step of detecting radiation to thereby acquire a datum on a distribution and/or an amount of expression of the hM4D or the hM3D in the brain can be achieved by using an imaging device PET. Specifically, the hM4D or the hM3D is expressed in advance in a live subject, and then a composition including the compound represented by Formula (III) or a pharmaceutically acceptable salt or solvate thereof is administered to the live subject and is allowed to pass through the blood-brain barrier (BBB) into the brain. The live subject is mounted in the PET device and continuously imaged by radiation emitted from the brain over a predetermined period.

The PET device used herein does not need to be special and may be common PET devices, that is, PET devices with multi-ring arrangement using a simultaneous calculation method. The devices may be capable of acquiring data in a two-dimensional (2D) or three-dimensional (3D) manner and may be operated with in-plane or axial mechanical scanning. Furthermore, the devices may also be devices capable of partially acquiring data, called micro-PET.

The compositions including the compounds can specifically bind to an hM4D or an hM3D produced by expressing a gene, which has been introduced into a cell in the brain of a live animal subject, in the cell. In this case, the compositions have a higher binding property for the hM4D or the hM3D, but have a lower binding property for endogenous substances such as wild-type receptors. Therefore, the compositions have high selectivity for the hM4D or the hM3D, leading to high-contrast images.

Therefore, this imaging method allows visualization of a site at which the gene encoding an hM4D or an hM3D is expressed and accurate display of a position of the site in the brain.

Furthermore, the compositions including these compounds emit radiation with high signal intensity, so that the hM4D or the hM3D can be quantitatively calculated from the resultant image.

### (8. Method for imaging change in brain activity associated with modulation of activity of hM4D- or hM3D-expressing cell in brain)

The present invention provides a method for imaging change in a brain activity of a live animal subject, the method comprising: introducing a gene encoding a mutated human M4 muscarinic acetylcholine receptor (hM4D) or a mutated human M3 muscarinic acetylcholine receptor (hM3D) into a cell in a brain of the live animal subject; administering the compound represented by Formula (III) that selectively binds to the hM4D or the hM3D or a pharmaceutically acceptable salt or solvate thereof to the live animal subject;
allowing the compound or a pharmaceutically acceptable salt or solvate thereof to migrate into the brain to thereby allow the compound or a pharmaceutically acceptable salt or solvate thereof to selectively bind to the hM4D or the hM3D expressed by the gene; and then
acquiring an image of the brain activity targeting the brain of the live animal subject to thereby acquire a datum on change in the brain activity associated with modulation of an activity of an hM4D- or hM3D-expressing cell in the brain. Here, the composition may be included in a pharmaceutically acceptable carrier, as mentioned in (4. Composition).

The image of the brain activity may be acquired by various methods. For example, glucose positron emission tomography which is a method for measuring the brain activity using a glucose metabolism as an index, [¹⁵O]H2O-PET which is a method for measuring the brain activity using a cerebral blood flow as an index, functional magnetic resonance (fMRI), functional near-infrared spectroscopy (fNIRS), endogenous optical measurement, or the like is available. As a method for visualizing an electrical activity of a nerve cell, electroencephalography (brain wave), magnetoencephalography (MEG), a voltage-sensitive dye imaging method (VSDI), or the like is available.

Specifically, in the glucose positron emission tomography, the hM4D or the hM3D is expressed in advance in a live subject, a composition including the compound represented by Formula (III) or a pharmaceutically acceptable salt or solvate thereof is administered to the live subject, the composition is allowed to selectively bind to the hM4D or the hM3D, a composition including a nuclide for glucose positron emission tomography is administered to the live subject, and then the nuclide is allowed to migrate into the brain. The live subject is mounted in the PET device and continuously imaged by radiation emitted from the brain over a predetermined period. When the glucose metabolism is active in hM4D- or hM3D-expressing cells, the nuclide for glucose positron emission tomography is consumed in a large amount. Meanwhile, when there is almost no glucose metabolism, almost no nuclide for glucose positron emission tomography is consumed. Therefore, a datum on a distribution and/or a metabolism amount of the glucose metabolism in an hM4D- or hM3D-expressed region can be acquired by measuring a distribution and/or a dose of radiation emitted from the nuclide for glucose positron emission tomography in the brain.

The nuclide for glucose positron emission tomography used herein may be known nuclides for glucose positron emission tomography and, for example, [¹⁸F]fluorodeoxyglucose (¹⁸F-FDG) may be suitably used. Furthermore, the nuclide may be used as a composition including saline. The composition may also include D-mannitol.

The PET device used herein does not need to be special and may be common PET devices, that is, PET devices with multi-ring arrangement using a simultaneous calculation method. The devices may be capable of acquiring data in a two-dimensional (2D) or three-dimensional (3D) manner and may be operated with in-plane or axial mechanical scanning. Furthermore, the devices may also be devices capable of partially acquiring data, called micro-PET.

The compound represented by Formula (III) can specifically bind to an hM4D or an hM3D produced by expressing a gene, which has been introduced into a cell in the brain of a live animal subject, in the cell.

In this case, the compound has a higher binding property for the hM4D or the hM3D, but has a lower binding property for endogenous substances such as wild-type receptors, resulting in a binding state in which selectivity for the hM4D or the hM3D is high.

Therefore, this method for imaging a glucose metabolism in the brain allows visualization of glucose metabolism distribution in a state in which the compound represented by Formula (III) is allowed to bind to an hM4D- or hM3D-expressed site, and high-precision measurement of a metabolism amount.

Furthermore, a datum on a tendency that the glucose metabolism is increased, decreased, or unchanged as a result of binding of the compound represented by Formula (III) to the hM4D- or hM3D-expressed site can also be acquired.

Furthermore, an increase or a decrease of the glucose metabolism as a result of binding of the compound represented by Formula (III) can be quantitatively obtained by measuring an amount of the glucose metabolism at the hM4D- or hM3D-expressed site and then comparing it with an amount of the glucose metabolism measured by the imaging method using the composition according to the present invention.

### (9. Agonist/Antagonist)

The present invention provides an antagonist or an agonist including a substance that selectively binds to a mutated human M4 muscarinic acetylcholine receptor (hM4D) or a mutated human M3 muscarinic acetylcholine receptor (hM3D), which has been introduced into a cell in the brain of a live animal subject, the antagonist or the agonist being the compound represented by Formula (III) or a pharmaceutically acceptable salt or solvate thereof. Here, one or more atoms in the Formula (III) are or are not radioisotopes of the atom or atoms.

In one embodiment, 11-(piperazine-1-yl)-5H-dibenzo[b,e][1,4]diazepine and 6-(4-methylpiperazine-1-yl)-11H-benzo[c][1]benzazepine may be excluded. Furthermore, in one embodiment, a compound in which one or more atoms in 11-(piperazine-1-yl)-5H-dibenzo[b,e][1,4]diazepine and 6-(4-methylpiperazine-1-yl)-11H-benzo[c][1]benzazepine are radioisotopes of the atom or atoms may be excluded.

For example, the compound represented by Formula (III) in which X is imino, R² is hydrogen, and R³ is methyl behaves as an agonist selectively acting on the mutated human M3 muscarinic acetylcholine receptor (hM3D) whether the compound is radiolabeled or not. Therefore, the present invention can provide an agonist especially when the mutated receptor is the mutated human M3 muscarinic acetylcholine receptor (hM3D).

Furthermore, the compound represented by Formula (III) in which X is imino, R² is hydrogen, and R³ is methyl behaves as an agonist selectively acting on the mutated human M4 muscarinic acetylcholine receptor (hM4D) whether the compound is radiolabeled or not. Therefore, an agonist can be provided when the mutated receptor is the mutated human M4 muscarinic acetylcholine receptor (hM4D).

### (10. Pharmaceutical)

The present invention provides a pharmaceutical including a substance that selectively binds to a mutated human M4 muscarinic acetylcholine receptor (hM4D) or a mutated human M3 muscarinic acetylcholine receptor (hM3D), which has been introduced into a cell in a brain of a live primate subject, the substance being the compound represented by Formula (IV) or a pharmaceutically acceptable salt or solvate.

As mentioned above, the compound represented by Formula (III) has a property of selectively acting on the mutated human M3 muscarinic acetylcholine receptor (hM3D) and the mutated human M4 muscarinic acetylcholine receptor (hM4D). Research on the brain of a live primate subject has proceeded focusing on this point and it has been found that a pharmaceutical which can treat a disease can be provided by introducing a gene for expressing the mutated human M3 muscarinic acetylcholine receptor (hM3D) or the mutated human M4 muscarinic acetylcholine receptor (hM4D) in a site involved in the disease in the brain, expressing the hM3D or the hM4D, and then administering the compound represented by Formula (IV) or a pharmaceutically acceptable salt or solvate thereof.

In one embodiment, 11-(piperazine-1-yl)-5H-dibenzo[b,e][1,4]diazepine may be excluded.

For example, the pharmaceutical is used for mental diseases, neurodegenerative diseases, cognitive/memory impairments, or sleep disorders.

Specific examples of the mental diseases include depression, major depression, bipolar depression, dysthymic disorder, affective disorder, recurrent depression, postpartum depression, stress disorder, depressive symptom, mania, anxiety, generalized anxiety disorder, anxiety syndrome, panic disorder, phobia, social phobia, social anxiety disorder, obsessive-compulsive disorder, posttraumatic stress syndrome, posttraumatic stress disorder, Tourette syndrome, autism, fragile X syndrome, Rett syndrome, adjustment disorder, bipolar disorder, neurosis, schizophrenia, chronic fatigue syndrome, anxiety neurosis, obsessional neurosis, panic disorder, epilepsy, nervous erethism, attention deficit hyperactivity disorder, psychotic major depression, refractory major depression, refractory depression, and the like.

Specific examples of the neurodegenerative diseases include Alzheimer's disease, senile dementia of Alzheimer type, Huntington's chorea, multi-infarct dementia, frontotemporal dementia, frontotemporal dementia of Parkinson type, progressive supranuclear palsy, Pick's syndrome, Niemann-Pick syndrome, corticobasal degeneration, Down's syndrome, defective dementia, Lewy body dementia, amyotrophic lateral sclerosis, motoneurogenic disease, Creutzfeldt-Jakob disease, cerebral palsy, progressive supranuclear palsy, multiple sclerosis, and the like.

Specific examples of the cognitive/memory impairments include age-related memory impairment, senile dementia, and the like.

Specific examples of the sleep disorders include intrinsic sleep disorder, extrinsic sleep disorder, circadian rhythm disorder, parasomnia, sleep disorder associated with medical or mental disorders, stress insomnia, insomnia, insomniac neurosis, sleep apnea syndrome, and the like. Additionally, the pharmaceutical is also used for traumatic brain injury, stroke, anorexia nervosa, eating disorder, anorexia nervosa, bulimia, other eating disorders, alcoholism, alcohol abuse, alcohol amnestic disorder, alcohol paranoia, alcoholophilia, alcohol withdrawal, alcoholic psychosis, alcoholism, alcoholic jealousy, alcoholic mania, alcoholic mental disorder, alcoholic psychosis, pharmacophilia, pharmacophobia, pharmacomania, drug withdrawal, migraine, stress headache, tension headache, diabetic neuropathy, obesity, diabetes, myospasm, Meniere's disease, dysautonomia, alopecia, glaucoma, deafness, hypertension, heart disease, tachycardia, congestive heart failure, hyperpnea, bronchial asthma, apnea, sudden infant death syndrome, inflammatory disease, allergic disease, impotence, menopausal symptom, sterility, cancer, immunologic deficiency syndrome due to HIV infection, cerebrospinal meningitis, acromegaly, incontinence, metabolic syndrome, osteoporosis, peptic ulcer, irritable bowel syndrome, inflammatory bowel disease, ulcerative colitis, Crohn's disease, stress dyspepsia, nervous vomiting, peptic ulcer, diarrhea, constipation, postoperative ileus, anesthetic, traumatic injury, or respiratory depression due to a neurodegenerative disease, and the like.

Note that the compound represented by Formula (IV) or a pharmaceutically acceptable salt or solvate thereof may be used in combination with other active components.

Furthermore, when used in the pharmaceutical, the compound represented by Formula (IV) or a pharmaceutically acceptable salt or solvate thereof may be formulated into a pharmaceutical formulation such as oral agents (tablets, capsules, powders, granules, subtle granules, pills, suspensions, emulsions, solutions, syrups, etc.), injections, eye-drops, or the like by incorporating a variety of pharmaceutical additives such as excipients, binders, disintegrants, collapse suppression agents, anticaking/antiadhesive agents, lubricants, absorption/adsorption vehicles, solvents, expanders, tonicity agents, solubilization agents, emulsifying agents, suspending agents, thickeners, coating agents, absorbefacients, gelatinizing/coagulation accelerators, photostabilizers, preservatives, desiccants, emulsion/suspension/dispersion stabilizers, anti-coloring agents, deoxidants/antioxidants, correctives, colorants, foaming agents, antifoaming agents, soothing agents, antistatic agents, buffering/pH adjusting agents, or the like.

A method for administering the formulation is not particularly limited, but is appropriately determined based on a dosage form; the age, sex, and other conditions of a subject; and the extent of a symptom in a patient.

Furthermore, as mentioned above, the compound represented by Formula (IV) can treat the disease involving an M3 muscarinic acetylcholine receptor or an M4 muscarinic acetylcholine receptor and, therefore, can provide a companion diagnostic agent for treating or preventing the disease. The companion diagnostic agent for treating the disease, as used herein, means a diagnostic agent for assessing whether the disease is expected to be treated when the disease is ascertained. Furthermore, the companion diagnostic agent for preventing the disease, as used herein, means a diagnostic agent for presuming future disease progress (prognosis) or assessing whether the disease is expected to be suppressed from further progressing when the disease is ascertained.

In the case of the companion diagnostic agent, one or more atoms in the compound represented by Formula (IV) may or may not be radioisotopes of the atom or atoms.

In one embodiment, 11-(piperazine-1-yl)-5H-dibenzo[b,e][1,4]diazepine may be excluded. Furthermore, in one embodiment, a compound in which one or more atoms in 11-(piperazine-1-yl)-5H-dibenzo[b,e][1,4]diazepine are radioisotopes of the atom or atoms may be excluded.

### (11. Method for imaging axon terminal of nerve cell across a plurality of regions)

The present invention provides a method for imaging a nerve cell across a plurality of regions in a brain of a live animal subject, the method comprising: introducing a gene encoding a mutated human M4 muscarinic acetylcholine receptor (hM4D) or a mutated human M3 muscarinic acetylcholine receptor (hM3D) into a first region in the nerve cell when a cell body including a dendrite belongs to the first region, but a axon terminal of the nerve cell belongs to a region different from the first region; administering a radiolabeled substance that selectively binds to the hM4D or the hM3D to the live animal subject;
allowing the substance to migrate into the brain to thereby allow the substance to selectively bind to the hM4D or the hM3D; and detecting radiation emitted from the substance selectively binding to the hM4D or the hM3D to thereby acquire a datum on a distribution and/or an amount of expression of the hM4D or the hM3D in the axon terminal.

It is said that there are 100 billion or more nerve cells (neurons) in the central nervous system composed of the brain and the spinal cord. The neuron includes an interneuron having a relatively short axon, localized to a specific site, and communicating with only an adjacent neuron; and a projection neuron having a relatively long axon and communicating with other regions. The neuron is a single cell and one neuron has one axon. The axon has various lengths ranging from several micrometers to one meter. The neural circuit is very complex and it is a brain-scientific challenge to elucidate which regions the projection neuron communicates therebetween.

The present inventors have conducted intensive studies, and as a result, have found that, for a nerve cell across a plurality of regions in a brain of a live animal subject, a datum on a distribution and/or an amount of expression of an hM4D or an hM3D at axon terminals can be acquired by introducing a gene encoding a mutated human M4 muscarinic acetylcholine receptor (hM4D) or a mutated human M3 muscarinic acetylcholine receptor (hM3D) into a first region in the cell in the brain of the live animal subject when a cell body constituting the nerve cell and including a dendrite belongs to the first region, but the axon terminal of the nerve cell belongs to a region different from the first region; administering a radiolabeled substance that selectively binds to the hM4D or the hM3D to the live animal subject; allowing the substance to migrate into the brain to thereby allow the substance to selectively bind to the hM4D or the hM3D expressed by the gene; and detecting radiation emitted from the substance selectively binding to the hM4D or the hM3D in the brain.

A nerve cell (neuron) in the present invention will be described with reference to microscale (microscopic) and mesoscale (mesoscopic) drawings.

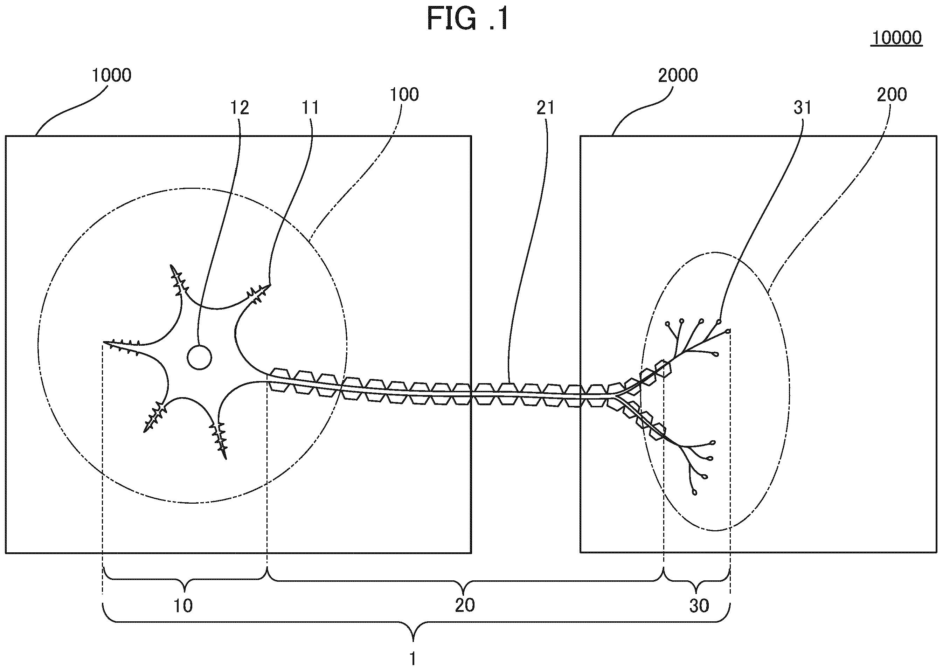

Fig. 1 is a microscale drawing showing a nerve cell (neuron) for describing the present invention. Fig. 1 shows one nerve cell (neuron) 1 present in a brain 10000. The nerve cell 1 is a projection neuron, both ends of the nerve cell 1 are in regions different from each other, as a result, the nerve cell 1 is across a plurality of regions. Specifically, the nerve cell 1 includes a cell body (or soma) 10, an axon 20, and an axon collateral (or telodendrion) 30.

The cell body 10 includes a dendrite 11. The cell body 10 may or may not include a nucleus 12, but Fig. 1 shows the cell body including the nucleus. The axon 20 includes a myelin sheath 21. The axon collaterals 30 include axon terminals 31.

The cell body 10 including the dendrite 11 belongs to a first region 1000. Furthermore, the axon collaterals 30 including the axon terminals 31 belong to a second region 2000. Here, the first region 1000 and the second region 2000 are different regions from each other and do not overlap with each other. Note that the region, as used herein, may be a region defined based on a brain structural classification, a tissue morphological classification, an anatomic classification, a cytological classification, a functional classification, a physical classification (including electrical properties), an information processing classification, brain atlas classification and the like. For example, the region may be a region based on the Broadmann area, which is also called a Broadmann brain map and one anatomic classification. In this case, the region is expressed as a region numbered with any of 1 to 52. Furthermore, the brain may be classified into regions numbered froom 1 to 83 with the emphasis on the functional classification. Furthermore, the brain may be classified into regions numbered from 1 to 180 with the emphasis on the cytological classification. In any case, importantly, the first region 1000 and the second region 2000 are different regions from each other and do not overlap with each other.

A composition including a gene encoding a mutated human M4 muscarinic acetylcholine receptor (hM4D) or a mutated human M3 muscarinic acetylcholine receptor (hM3D) is introduced into the first region 1000. Specifically, a liquid composition including the gene encoding an hM4D or an hM3D is injected by, for example, a microsyringe from the outside of the brain so as to reach the cell body 10 including the dendrite 11 in the nerve cell 1 of interest. An amount of injection is not particularly limited, but is an amount larger than that needed to express the gene, for example, 1 to 1000 µL in order for the gene to surely reach a predetermined nerve cell 1. Therefore, the injected composition occupies a spherical injection region 100 (region shown by an alternate long and two short dashes line) surrounding the cell body 10 including the dendrite 11 of the nerve cell 1 of interest. In other words, the gene encoding an hM4D or an hM3D is injected into the region 1000 and the cell body 10 including the dendrite 11 is included in the region 1000.

The gene encoding an hM4D or an hM3D is introduced into the cell body 10 including the dendrite 11. According to the amount of injection of the composition, the gene may also be introduced into a part of the axon 20 inescapably connected to the cell body 10, in addition to the cell body 10 including the dendrite 11. In any case, the injection region 100 is included in the first region 1000.

It takes a predetermined period to express the introduced gene throughout the nerve cell 1 of a live subject. For example, the period is 1 day or longer, 3 days or longer, 5 days or longer, 7 days or longer, 10 days or longer, 20 days or longer, 30 days or longer, 40 days or longer, 50 days or longer, 60 days or longer, 100 days or longer, 150 days or longer, 200 days or longer, 250 days or longer, 300 days or longer, 400 days or longer, or 500 days or longer. Expression of the gene results in producing the hM4D receptor or the hM3D receptor, which would be capable of being observed from the outside of an animal after the predetermined period.

Specifically, a radiolabeled substance that selectively binds to the hM4D or the hM3D is externally administered and allowed to migrate into the brain. This substance selectively binds to the hM4D or the hM3D expressed by the gene also in the axon terminals 31 which are projection targets of the nerve cell 1. As a result, the axon terminals 31 are radiolabeled, allowing molecular imaging. If a radiation intensity is converted to and expressed as brightness (luminance) upon imaging, the axon terminals 31 can be observed by light emitted therefrom along with information on morphology in principle. The axon terminals 31 emitting light are included in the second region 2000.

When the molecular imaging is PET imaging, a general-purpose PET device does not have a microscale spatial resolution (spatial resolving power) at present and therefore it is observed as if radiation is emitted from the entire axon collaterals 30 including the axon terminals 31 branched from the nerve cell 1. In other words, an emitting region 200 (shown by an alternate long and short dash line) including the axon terminals 31 is collectively observed.

That is, the emitting region 200 different from the first region into which the gene is introduced is observed. This reveals a region to which the nerve cell 1 having the dendrite 11 in the first region 100 is connected and projected, when a neurotransmission pathway is unknown.

Furthermore, a datum on a distribution and/or an amount of expression of the hM4D or the hM3D in the synaptic axon terminals 31 can be acquired.

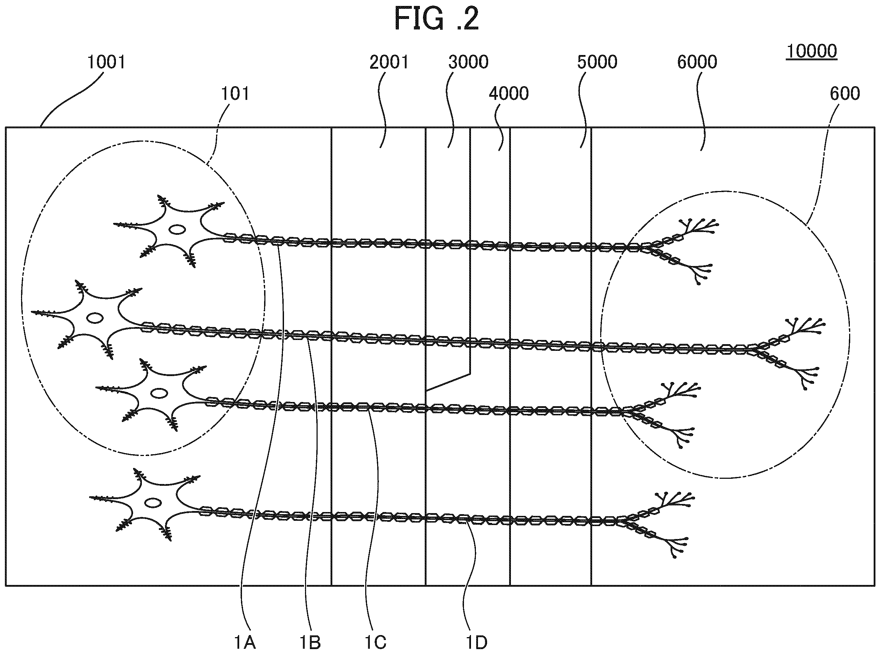

Fig. 2 is a mesoscale drawing showing a nerve cell for describing the present invention. Fig. 2 shows four nerve cells 1A to 1D present in a brain 10000, as one example. The nerve cells 1A to 1D are all projection neurons, both ends of each of the nerve cells 1A to 1D are in regions different from each other. As a result, each of the nerve cells 1A to 1D is across different regions.

Structural detail of the nerve cells 1A to 1D is the same as that of the nerve cell 1 in Fig. 1 and therefore description and illustration thereof are omitted. Cell bodies each including a dendrite in the nerve cells 1A to 1D all belong to a first region 1001. Axon collaterals each including axon terminals in the nerve cells 1A to 1D all belong to a sixth region 6000.

Here, the first region 1001 and the sixth region 6000 are different regions from each other and do not overlap with each other. In other words, the axon collaterals including the axon terminals belong to a region different from that of the cell body including the dendrite.

Note that, as shown in Fig. 2, a second region 2001, a third region 3000, a fourth region 4000, and a fifth region 5000 are present between the first region 1001 and the sixth region 6000 in brain atlas space. Considering each of the nerve cells, for the nerve cells 1A and 1B, the second region 2001, the third region 3000, the fourth region 4000, and the fifth region 5000 are present between the first region 1001 and the sixth region 6000.

For the nerve cells 1C and 1D, the second region 2001, the fourth region 4000, and the fifth region 5000 are present between the first region 1001 and the sixth region 6000 (the third region 3000 does not intervene therebetween).

Here, a liquid composition including the gene encoding an hM4D or an hM3D is injected by, for example, a microsyringe from the outside of the brain to the first region 1001 to thereby occupy an injection region 101(shown by an alternate long and two short dashes line). In this case, the injection region 101 is in the first region 1001. Considering each of the nerve cells, only the nerve cells 1A to 1C are in the injection region 101. In this case, the nerve cells 1A to 1C express the gene, but the nerve cell 1D does not express the gene.

Therefore, when a radiolabeled substance that selectively binds to hM4D or hM3D is externally administered after the gene is expressed to thereby migrate into the brain, only the axon terminals of the nerve cells 1A to 1C can be observed by molecular imaging. In the case of PET imaging, an emitting region 600 (shown by an alternate long and short dash line) is observed including the axon terminals of the nerve cells 1A to 1C, but not including the axon terminal of the nerve cell 1D lacking expression of the gene.

Furthermore, the nerve cells 1A and 1B are observed to emit light similar to the nerve cell 1C which does not pass through the third region and therefore there is no impact by other regions between the first region 1001 and the sixth region 6000 (regions other than the first region 1001 and the sixth region 6000).

Thus, when the composition including the gene is very accurately injected from the outside of the brain in an extremely small amount, a neurotransmission process can be elucidated for every nerve cell or a bundle of nerve cells.

Note that, upon imaging, an imaging time is not particularly limited. For example, the imaging may start immediately after the substance is administered and finish at any time. Furthermore, the imaging may start immediately after the substance is allowed to migrate into the brain and finish at any time.

Furthermore, the imaging may start after a predetermined period elapses from administration of the substance or transition of the substance into the brain and finish at any time. Thus, some of the substances which do not contribute to binding to the nerve cell 1 or other cells are washed away, resulting in easy observation of the axon terminals 31. Furthermore, noise is decreased when acquiring a datum on a distribution and/or an amount of expression of the hM4D or the hM3D in the axon terminals 31.

Here, the predetermined period may be set for each live animal subject of interest. For example, the predetermined period may be 10 minutes or more, 20 minutes or more, 30 minutes or more, 40 minutes or more, 50 minutes or more, 60 minutes or more, 70 minutes or more immediately after the substance is administered. Furthermore, the imaging may be finished at any time, but when the imaging is PET imaging and the substance is [¹¹C], the imaging may be performed for 50 minutes or less, 60 minutes or less, 70 minutes or less, 80 minutes or less, 90 minutes or less, 100 minutes or less, 110 minutes or less, 120 minutes or less, or 130 minutes or less in view of half-life. Note that, in order to clearly observe the axon terminals 31, a position and an angle of a cross-section of an image are adjusted to create an image drawing so that the axon terminals 31 are exposed on the cross-section.

The imaging of the axon terminals 31 in the brain after the composition including the gene encoding an hM4D or an hM3D is introduced into and expressed in the brain has been described. Here, a region in which expression of the gene is increased is more easily identified when the imaging is performed in advance before the gene is introduced since a differential image before and after the gene is introduced can be electronically created.

### (12. Composition for imaging axon terminals of nerve cell across a plurality of regions)

The present invention provides a composition for imaging a axon terminal of a nerve cell, into which a gene encoding a mutated human M4 muscarinic acetylcholine receptor (hM4D) or a mutated human M3 muscarinic acetylcholine receptor (hM3D) is introduced, in a brain of a live animal subject, the composition including:
a radiolabeled dibenzoazepine derivative or a pharmaceutically acceptable salt or solvate thereof.

Here, the dibenzoazepine derivative means a compound having a dibenzoazepine skeleton or a compound in which a dibenzoazepine skeleton is substituted with an appropriate substituent. Here, examples of the compound in which a dibenzoazepine skeleton is substituted with an appropriate substituent include a compound having a dibenzodiazepine skeleton or a compound having a dibenzothiazepine skeleton.

Examples of diazepine include 1,2-diazepine, 1,3-diazepine, or 1,4-diazepine.

Examples of thiazepine include 1,3-thiazepine or 1,4-thiazepine.

Examples of the dibenzoazepine derivative include a dibenzo[b,e]azepine derivative, a dibenzo[b,e][1,4]diazepine derivative, and a dibenzo[b,e][1,4]thiazepine derivative.

These dibenzoazepine derivatives may be further substituted with an appropriate substituent. Examples of the appropriate substituent include alkyl, acyl, aryl, a nitrogen-containing heterocycle, a sulfur-containing heterocycle, an oxygen-containing heterocycle, and halogen.

The compound may be formed into a composition by incorporating it in a pharmaceutically acceptable carrier, as mentioned in (4. Composition). Furthermore, when the imaging is PET imaging, one or more atoms in the compound are radioisotopes of the atom or atoms. As one example of the radioisotopes, a carbon atom may be ¹¹C and a nitrogen atom may be ¹³N.

One example of substitution of the dibenzoazepine derivative with the nitrogen-containing heterocycle is substitution with piperazine. Examples thereof include a compound in which the dibenzoazepine skeleton is substituted with piperazine at position 11. For example, the compound may be 11-piperazino-5H-dibenzo[b,e][1,4]diazepine.

The nitrogen-containing heterocycle substituted for the dibenzodiazepine skeleton may be further substituted with an appropriate substituent. As one example, in the case of 11-piperazino-5H-dibenzo[b,e][1,4]diazepine, a nitrogen on the piperazine skeleton may be substituted with an alkyl group.

One embodiment of the present invention is the compound represented by Formula (I) in which the alkyl substituent is a methyl group. Here, one or more atoms in the compound represented by Formula (I) are radioisotopes of the atom or atoms. As one example of the radioisotopes, a carbon atom may be ¹¹C. An additional example is the compound represented by Formula (II).

Furthermore, when halogen in the dibenzoazepine skeleton may be substituted, the halogen may be fluoro (-F), chloro (-Cl), bromo (-Br), or iodo (-I).

Another embodiment of the present invention is a compound represented by Formula (VI) in which the halogen substituent is chloro. 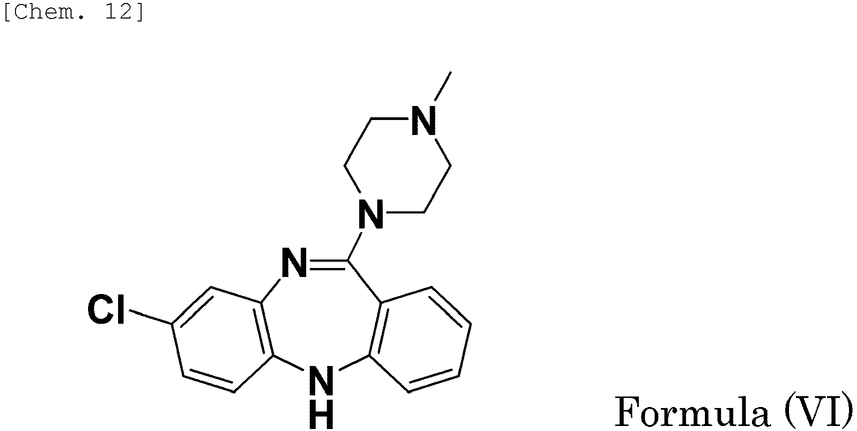 Here, one or more atoms in the compound represented by Formula (VI) are radioisotopes of the atom or atoms. As one example of the radioisotopes, a carbon atom may be ¹¹C.

### EXAMPLES

Examples will be described. The following examples are described to promote a better understanding of the claims and are not intended to limit the claims. Note that the following animal experiments were conducted under the approval of the Institutional Animal Care and Use Committee of National Institutes for Quantum and Radiological Science and Technology.

### Example 1

The head of Japanese macaque (male/5.5 kg) was fixed to a stereotaxic instrument under general anesthesia, and 6 µL of an adeno-associated virus (AAV) vector (AAV2-CMV-hM4D) for expressing an hM4D receptor in a nerve cell-specific manner was injected for 12 minutes by a Hamilton syringe (10 µL) into a middle portion of the right putamen read from a magnetic resonance imaging (MRI) image taken in advance. Note that, here, the CMV was a promotor for gene expression.

On the day 427 days after the injection, a compound represented by Formula (II) (hereinafter may be referred to as [¹¹C]C22b) (364 MBq) serving as a radiolabeled tracer was intravenously administered under isoflurane anesthesia and subjected to PET scanning for 90 minutes.

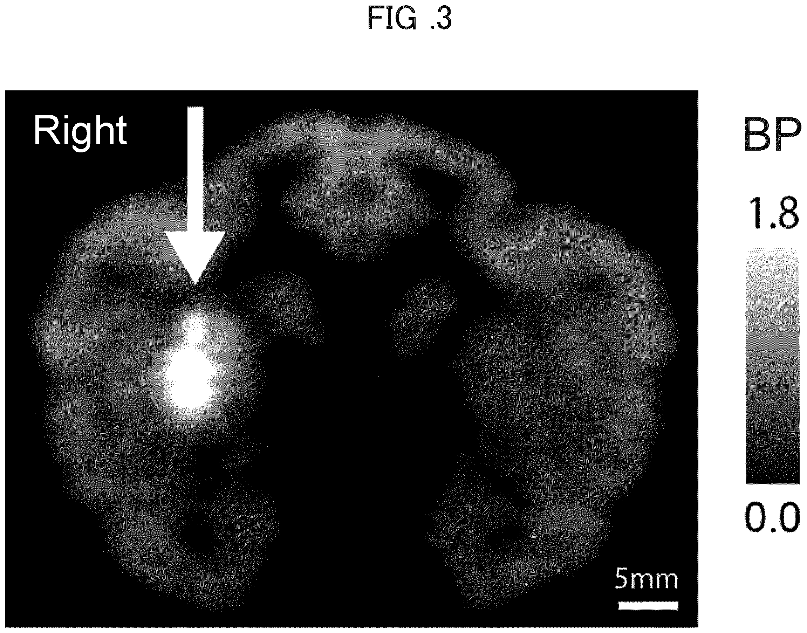

An image of a ligand binding potential was created from PET data integrated for 90 minutes using the cerebellum as a reference region (the binding potential (BP) in the cerebellum was determined as 1). The thus-created image is shown in Fig. 3.

A portion shown by an arrow in Fig. 3 represents the right putamen into which the AAV vector has been injected. As shown in Fig. 3, a region having a high binding potential to [¹¹C]C22b was found in the right putamen into which the AAV vector had been injected. When the binding potential was converted to numerical form, the binding potential in a region opposite to the site into which the AAV vector had been injected, serving as a control region (corresponding to the left putamen) was 0.28, while the binding potential in the right putamen into which the AAV vector had been injected was 3.07, in other words, a significant binding potential 11 times that of a non-injection region was shown. Thus, the hM4D receptor was confirmed to be expressed.

### Example 2, and Comparative Example 1

The [¹¹C]C22b (Example 2) and [¹¹C]clozapine ([¹¹C]CLZ, Comparative Example 1) were compared for a property of visualizing hM4D expressed in the Japanese macaque used in Example 1. The hM4D receptor was expressed in the same manner as in Example 1.

An average image integrated from 30 to 90 minutes after administration of a tracer was created based on a standardized uptake value (SUV) and an amount of uptake in the brain was calculated from PET imaging taken from 30 to 90 minutes after the administration in the frontal plane including the right putamen in which the hM4D was expressed.

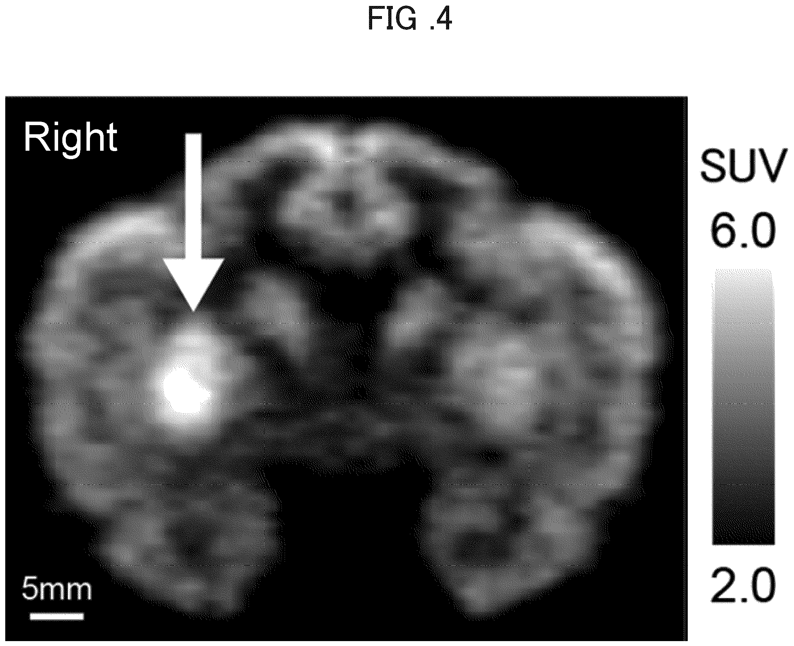

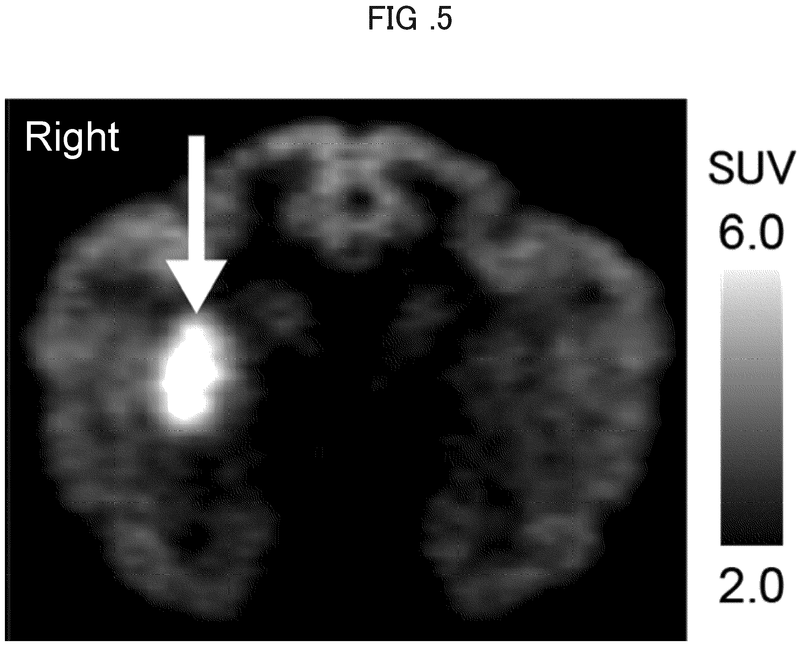

Figs. 4 and 5 are the average images from 30 to 90 minutes after the administration, in the case of using [¹¹C] clozapine (Fig. 4) and [¹¹C]C22b (Fig. 5) as the tracer.

Table 1 shows amounts of uptake of the tracers (amounts of the tracers per volume (g/cc)) in the right putamen region into which the AAV vector has been injected and in an opposite region serving as a control region (corresponding to the left putamen). Selectivity, that is, a ratio of an amount of the tracer per volume of an expressed region to an amount of the tracer per volume of an unexpressed region was 1.25 for the [¹¹C]clozapine, while the selectivity was high of 2.31 for [¹¹C]C22b.

The [¹¹C]C22b was incorporated into the hM4D expressed region in a larger amount, but into the unexpressed control region in a smaller amount than [¹¹C]clozapine, indicating high selectivity for designer receptors.

**[Table 1]**

| | Whole brain g/cc | hM4D-expressed region g/cc | Control region (hM4D-unexpressed region) g/cc | Selectivity (expressed region / unexpressed region) no unit of quantity |
|---|---|---|---|---|
| [¹¹C]C22b | 2.47 | 6.32 | 2.74 | 2.31 |
| [¹¹C]clozapine | 3.24 | 5.73 | 4.57 | 1.25 |

### Example 3

The head of Macaca mulatta (male/4.2 kg) was fixed to a stereotaxic instrument under general anesthesia, 3 µL of an adeno-associated virus (AAV) vector (AAV2-CMV-hM3D) for expressing an hM3D receptor in a nerve cell-specific manner was injected for 12 minutes by a Hamilton syringe (10 µL) into a middle portion of the left amygdala read from a magnetic resonance imaging (MRI) image taken in advance. Note that, here, the CMV was a promotor for gene expression.

On the day 57 days after the injection, the [¹¹C]C22b (277 MBq) serving as a radiolabeled tracer was intravenously administered under isoflurane anesthesia and subjected to PET scanning for 90 minutes.

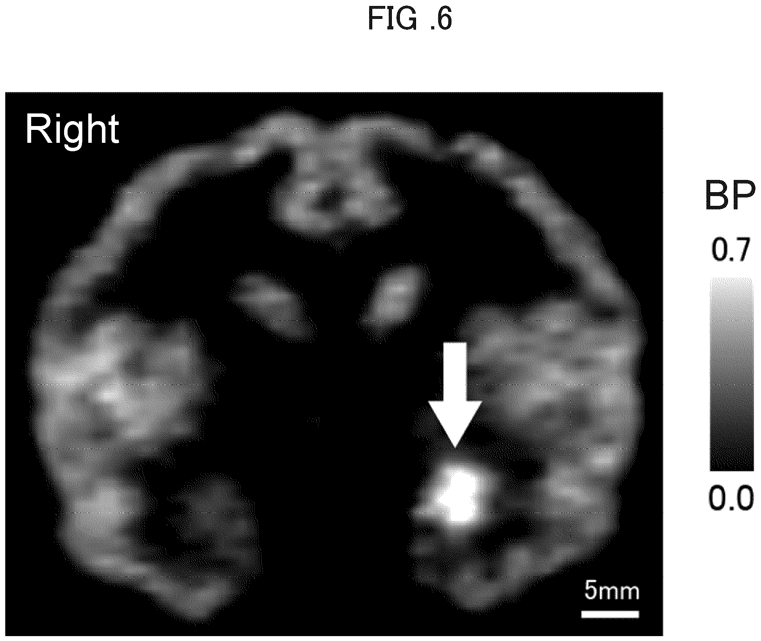

An image of a ligand binding potential was created from PET data integrated for 90 minutes using the cerebellum as a reference region (the binding potential (BP) of the cerebellum was determined as 1). The thus-created image is shown in Fig. 6. A portion shown by an arrow in Fig. 6 represents the left amygdala into which the AAV vector has been injected. As shown in Fig. 6, a region having a high binding potential to [¹¹C]C22b was found in the left amygdala into which the AAV vector had been injected.

When the binding potential is converted to numerical form, the binding potential in a region opposite to the site into which the AAV vector had been injected, serving as a control region (corresponding to the right amygdala) was 0.31, while the binding potential in the left amygdala into which the AAV vector had been injected was 1.05, in other words, a significant binding potential 3.4 times that of a non-injection region was shown. Thus, the hM3D receptor was confirmed to be expressed.

Table 2 shows amounts of uptake of the tracers (amounts of the tracers per volume (g/cc)) in the region into which the AAV vector had been injected and in an opposite region serving as a control region. The selectivity, that is, a ratio of an amount of the tracer per volume of an expressed region to an amount of the tracer per volume of an unexpressed region was a high value of 1.45.

**[Table 2]**

| | Whole brain g/cc | hM3D-expressed region g/cc | Control region (hM3D-unexpressed region) g/cc | Selectivity (expressed region / unexpressed region) no unit of quantity |
|---|---|---|---|---|
| [¹¹C]C22b | 2.42 | 3.77 | 2.60 | 1.45 |

### Example 4

On the day 65 days after the AAV vector was injected, a C22b compound (0.1 mg/kg) was intravenously administered to the same Macaca mulatta as in Example 3 under isoflurane anesthesia, 1 minute later, ¹⁸F-FDG (263 MBq) was intravenously administered, and then subjected to PET scanning for 90 minutes. Note that, here, the C22b compound was HY-42110 (manufactured by MedChem Express) which was the compound represented by Formula (III) in which R¹ was methyl, X was imino, and R² was hydrogen.

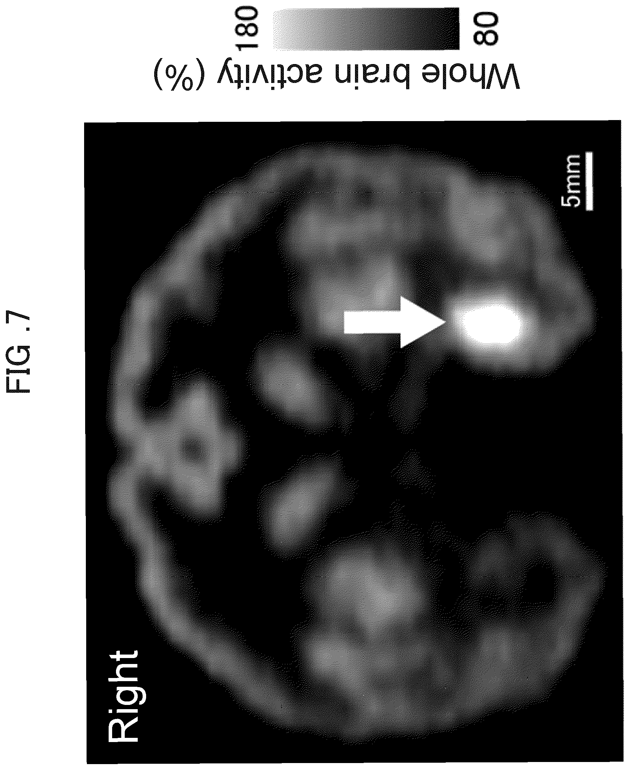

A glucose metabolism image (whole Brain Activity) when an average in the whole brain was determined as 100% was created from PET data integrated for 90 minutes. The thus-created image is shown in Fig. 7.

As shown in Fig. 7, high glucose metabolism was found in the left amygdala (arrowed) into which the vector had been injected. When the glucose metabolism is converted to numerical form, the left amygdala into which the AAV vector had been injected had a significantly activated glucose metabolism 1.88 times that of a region opposite to the region into which the AAV vector had been injected, serving as a control region (corresponding to the right amygdala) For this reason, it was confirmed that the glucose metabolism was activated when the hM3D receptor was expressed, in other words, the hM3D receptor acted as an agonist.

Then, the AAV vector was injected into Macaca mulatta different from in Example 3 (male, 3 kg) in the same manner as in Example 3. On the day 70 days after, a C22b compound solution (1 µg/kg) or a solvent was intravenously administered under isoflurane anesthesia, 1 minute later, ¹⁸F-FDG (224 to 286 MBq) was intravenously administered, and then subjected to PET scanning for 120 minutes. The process was performed four times for each intravenous dose. Note that the solvent was the same as a solvent used for the C22b compound solution (solvent: 0.1 mL/kg of saline containing 1% dimethyl sulfoxide and 5% Tween 20 (surfactant)).

A glucose metabolism image (whole brain activity) when an average of the whole brain was determined as 100% was created from PET data integrated from 30 minutes to 60 minutes after the administration. Then, a site in which the C22b administration caused a statistically significant change compared to a solvent administration was analyzed by one-way repeated measures ANOVA using MATLAB (R2016a) and SPM12. A region in which a glucose metabolism activity was significantly high was found in the left amygdala into which the AAV vector had been introduced (uncorrected p < 0.001).

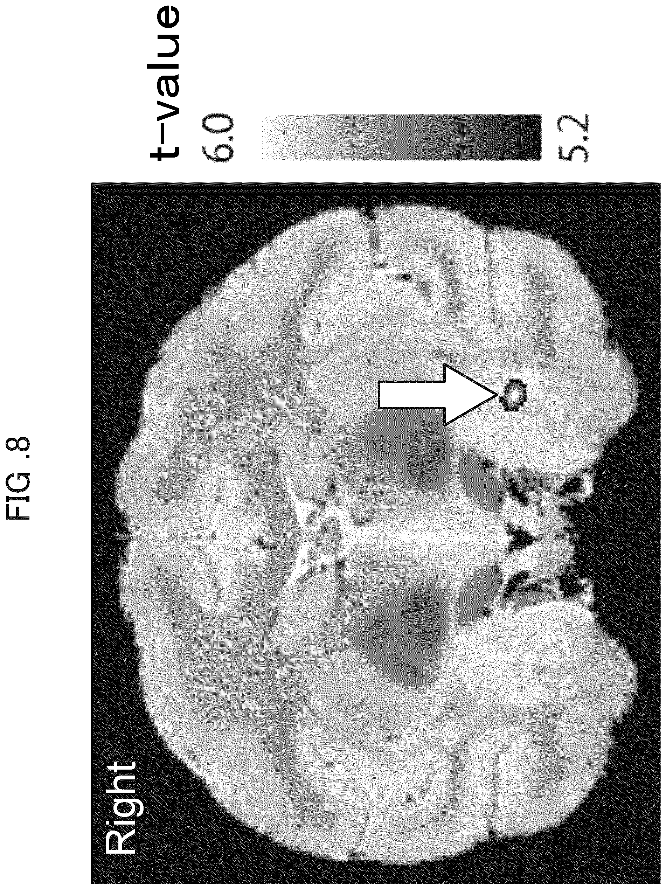

Furthermore, Fig. 8 is an image in which an image analyzed by one-way repeated measures ANOVA for the left amygdala (corresponding to the point of the arrow) is overlaid on an image of the brain structure (whole). As shown in Fig. 8, a t-value for the AAV vector injected region was t > 5.2, confirming that the glucose metabolism activity was significant.

### Example 5

The substantia nigra pars reticulata, which was a projection target of a nerve cell in the right putamen, was observed in the Japanese macaque used in Example 1. Specifically, the [¹¹C]C22b was exogenously administered as a tracer in the same manner as in Example 1 and subjected to PET scanning for 90 minutes. After the PET scanning, an average image integrated from 30 to 90 minutes after administration of the tracer was created.

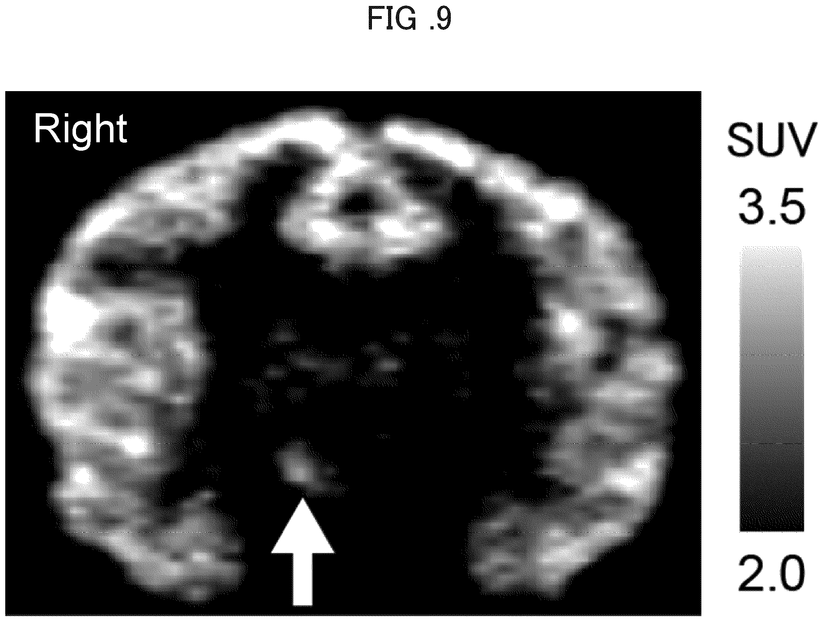

Fig. 9 shows the frontal plane including the substantia nigra pars reticulata of the thus-created image (SUV was expressed by dark and light shading). An arrow in this figure indicates the substantia nigra pars reticulata and therefore the hM4D was confirmed to be expressed in the axon terminal which is a projection target of the nerve cell.

### Example 6

The substantia nigra pars reticulata, which was a projection target of a nerve cell in the right putamen was observed in the Japanese macaque in the same manner as in Example 5. Specifically, the compound represented by Formula (V) ([¹¹C]C22b) was exogenously administered as a tracer and subjected to PET scanning for 90 minutes. After the PET scanning, an average image integrated from 30 to 90 minutes after administration of the tracer was created.

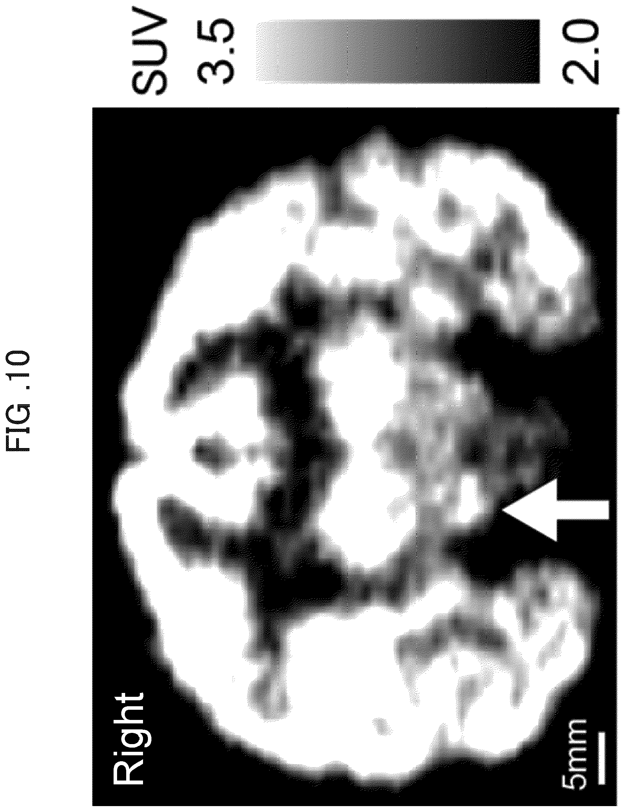

Fig. 10 shows the frontal plane including the substantia nigra pars reticulata of the thus-created image (SUV was expressed by dark and light shading).

An arrow in this figure indicates the substantia nigra pars reticulata and therefore the hM4D was confirmed to be expressed in the axon terminal which is a projection target of the nerve cell. However, unlike the case of [¹¹C]C22b, the compound represented by Formula (VI) ([¹¹C]8-chloro-11-(4-methylpiperazine-1-yl)-5H-dibenzo[b,e][1,4]diazepine) was also allowed to bind to nerve cells other than the nerve cell of interest or brain tissues to some extent. When a target nerve cell is desired to be more clearly identified or when high resolution is required, the [¹¹C]C22b is desirably used.

As shown by the above Examples, the compound of the present invention has both specificity and quantitativity for an hM4D receptor or an hM3D receptor, a designer receptor, and allows the hM4D receptor or the hM3D receptor expressed in an organ of a live animal subject to be imaged. Furthermore, the compound of the present invention can activate the hM4D receptor or the hM3D receptor.

Furthermore, the compound of the present invention can provide an agonist, an antagonist, a companion diagnostic agent, and a therapeutic agent for diseases involving the hM4D or the hM3D receptor.

Furthermore, the compound of the present invention allows the axon terminal, which is a projection target of a projection neuron in a live animal subject, to be imaged.

### EXPLANATION OF REFERENCE NUMERALS

- 1:: nerve cell
- 10:: cell body
- 11:: dendrite
- 12:: nucleus
- 20:: axon
- 21:: myelin sheath
- 30:: axon collateral
- 31:: axon terminal
- 100:: injection region
- 101:: injection region
- 200:: emitting region
- 600:: emitting region
- 1000:: first region
- 1001:: first region
- 2000:: second region
- 2001:: second region
- 3000:: third region
- 4000:: fourth region
- 5000:: fifth region
- 6000:: sixth region
- 10000:: brain

## Claims

1. A compound represented by Formula (I) or a pharmaceutically acceptable salt or solvate thereof: 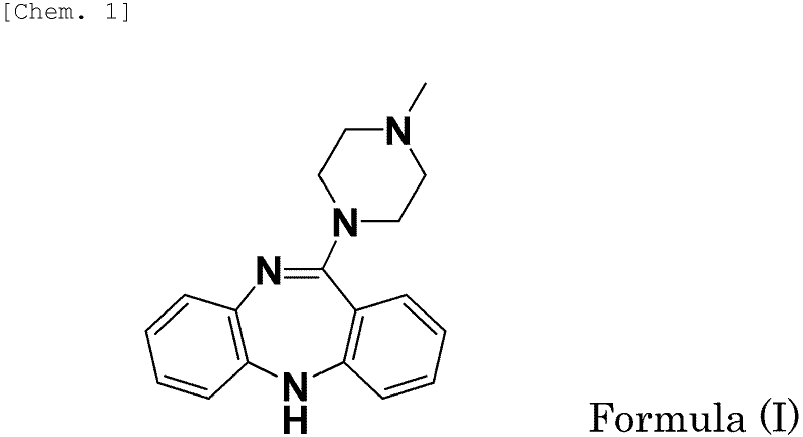 wherein one or more atoms are or are not radioisotopes of the atom or atoms.

2. The compound or a pharmaceutically acceptable salt or solvate thereof according to claim 1, wherein the compound is a compound represented by Formula (II):

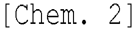

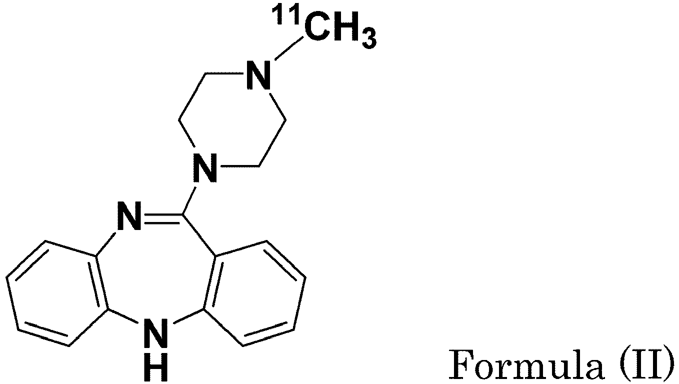

3. A composition comprising:
the compound or a pharmaceutically acceptable salt or solvate thereof according to claim 1 or 2.

4. A composition for imaging a mutated human M4 muscarinic acetylcholine receptor (hM4D) or a mutated human M3 muscarinic acetylcholine receptor (hM3D) produced by expressing a gene encoding the hM4D or the hM3D which has been introduced into a cell in a brain of a live animal subject, the composition comprising:
a compound represented by Formula (III) or a pharmaceutically acceptable salt or solvate thereof: 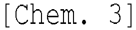 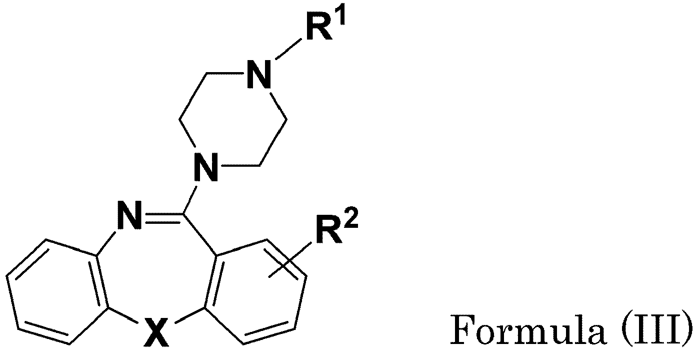
wherein R¹ represents hydrogen, alkyl, allyl, hydroxyalkyl, alkoxyalkyl, or alkoyloxyalkyl;
the alkyl having 1 to 6 carbon atoms, the hydroxyalkyl having 1 to 2 carbon atoms, and the alkoxyalkyl and the alkoyloxyalkyl having 1 to 5 carbon atoms;
X represents sulfur, sulfinyl, imino, methylene, or alkylimino;
the alkylimino having 1 to 6 carbon atoms;
R² represents hydrogen, halogen, hydroxy, trifluoromethyl, alkyl, alkoxy, alkylthio, nitro, amino, or aminosulfonyl;
the alkyl, the alkoxy, and the alkylthio having 1 to 5 carbon atoms; and
wherein one or more atoms are radioisotopes of the atom or atoms.

5. The composition according to claim 4, wherein R¹ represents alkyl.

6. The composition according to claim 5, wherein X represents imino and R² represents hydrogen.

7. A composition for imaging a mutated human M4 muscarinic acetylcholine receptor (hM4D) or a mutated human M3 muscarinic acetylcholine receptor (hM3D) produced by expressing a gene encoding the hM4D or the hM3D which has been introduced into a cell in a brain of a live animal subject, the composition comprising:
a dibenzodiazepine derivative or a pharmaceutically acceptable salt or solvate thereof,
the dibenzodiazepine derivative being radiolabeled, and
the derivative emitting radiation in a dose of 2 times or more for an hM4D-expressed site or 1.4 times or more for an hM3D-expressed site that of an unexpressed site as detected by imaging over a predetermined period from peripheral administration.

8. The composition according to claim 7, wherein the live animal subject is a live primate subject,
the predetermined period is 30 to 90 minutes, and
the dose to be detected is 6.3 g/cc or more for the hM4D-expressed site or 3.7 g/cc or more for the hM3D-expressed site when the dose is 2.5 g/cc or less for a whole brain comprising the unexpressed site as expressed as an index normalized to an amount of administration and a body weight.

9. A method for imaging an hM4D or an hM3D in a brain of a live animal subject, the method comprising:
acquiring data on a distribution and/or an amount of expression of the hM4D or the hM3D in the brain containing a cell having an introduced gene encoding a mutated human M4 muscarinic acetylcholine receptor (hM4D) or a mutated human M3 muscarinic acetylcholine receptor (hM3D)
in the live animal subject administred with a compound or a pharmaceutically acceptable salt or solvate thereof that selectively binds to the hM4D or the hM3D, the compound being represented by Formula (III): 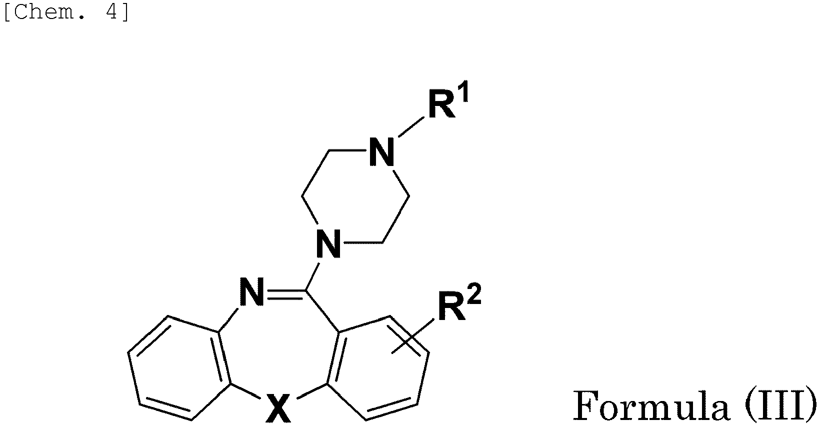
wherein R¹ represents hydrogen, alkyl, allyl, hydroxyalkyl, alkoxyalkyl, or alkoyloxyalkyl;
the alkyl having 1 to 6 carbon atoms, the hydroxyalkyl having 1 to 2 carbon atoms, and the alkoxyalkyl and the alkoyloxyalkyl having 1 to 5 carbon atoms;
X represents sulfur, sulfinyl, imino, methylene, or alkylimino;
the alkylimino having 1 to 6 carbon atoms;
R² represents hydrogen, halogen, hydroxy, trifluoromethyl, alkyl, alkoxy, alkylthio, nitro, amino, or aminosulfonyl;
the alkyl, the alkoxy, and the alkylthio having 1 to 5 carbon atoms; and
wherein one or more atoms are radioisotopes of the atom or atoms,
the compound or a pharmaceutically acceptable salt or solvate thereof being allowed to migrate into the brain to selectively bind to the hM4D or the hM3D expressed by the gene, wherein
the data is aquired by detecting radiation emitted from the compound or a pharmaceutically acceptable salt or solvate thereof selectively binding to the hM4D or the hM3D in the brain .

10. A method for imaging a brain activity of a live animal subject, the method comprising:
acquiring data on modulation of an activity of an hM4D- or hM3D-expressing cell in the brain containing a cell having an introduced gene encoding a mutated human M4 muscarinic acetylcholine receptor (hM4D) or a mutated human M3 muscarinic acetylcholine receptor (hM3D)
in the live animal subject administered with a compound or a pharmaceutically acceptable salt or solvate thereof that selectively binds to the hM4D or the hM3D, the compound being represented by Formula (III): 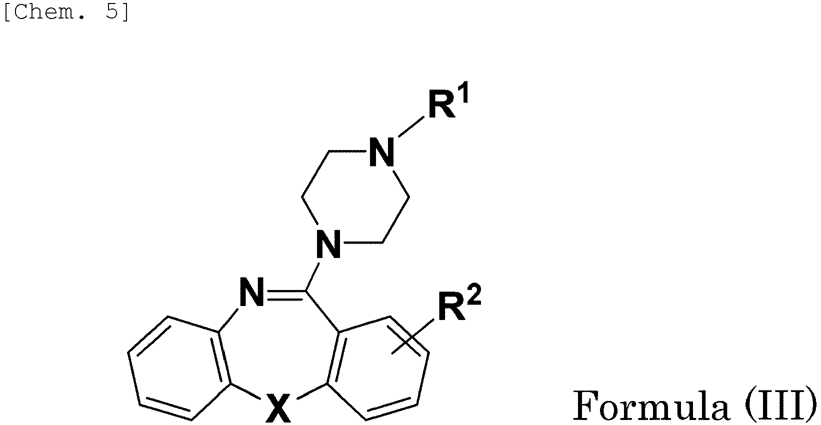
wherein R¹ represents hydrogen, alkyl, allyl, hydroxyalkyl, alkoxyalkyl, or alkoyloxyalkyl;
the alkyl having 1 to 6 carbon atoms, the hydroxyalkyl having 1 to 2 carbon atoms, and the alkoxyalkyl and the alkoyloxyalkyl having 1 to 5 carbon atoms;
X represents sulfur, sulfinyl, imino, methylene, or alkylimino;
the alkylimino having 1 to 6 carbon atoms;
R² represents hydrogen, halogen, hydroxy, trifluoromethyl, alkyl, alkoxy, alkylthio, nitro, amino, or aminosulfonyl;
the alkyl, the alkoxy, and the alkylthio having 1 to 5 carbon atoms,
the compound or a pharmaceutically acceptable salt or solvate thereof being allowed to migrate into the brain to selectively bind to the hM4D or the hM3D expressed by the gene.

11. An antagonist or an agonist comprising: a substance that selectively binds to a mutated human M4 muscarinic acetylcholine receptor (hM4D) or a mutated human M3 muscarinic acetylcholine receptor (hM3D) introduced into a cell in a brain of a live animal subject,
the substance being a compound represented by Formula (III) or a pharmaceutically acceptable salt or solvate thereof: 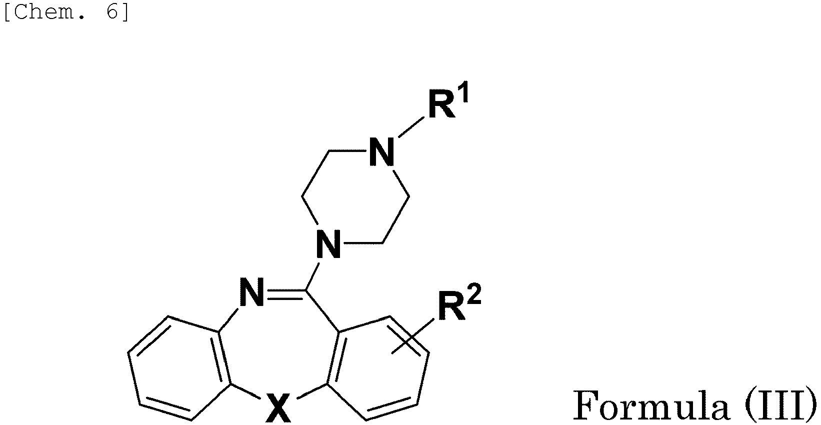
wherein R¹ represents hydrogen, alkyl, allyl, hydroxyalkyl, alkoxyalkyl, or alkoyloxyalkyl;
the alkyl having 1 to 6 carbon atoms, the hydroxyalkyl having 1 to 2 carbon atoms, and the alkoxyalkyl and the alkoyloxyalkyl having 1 to 5 carbon atoms;
X represents sulfur, sulfinyl, imino, methylene, or alkylimino;
the alkylimino having 1 to 6 carbon atoms;
R² represents hydrogen, halogen, hydroxy, trifluoromethyl, alkyl, alkoxy, alkylthio, nitro, amino, or aminosulfonyl;
the alkyl, the alkoxy, and the alkylthio having 1 to 5 carbon atoms; and
wherein one or more atoms are or are not radioisotopes of the atom or atoms.

12. The agonist according to claim 11, wherein the mutated receptor is the mutated human M3 muscarinic acetylcholine receptor (hM3D).

13. A pharmaceutical comprising:
a substance that selectively binds to a mutated human M4 muscarinic acetylcholine receptor (hM4D) or a mutated human M3 muscarinic acetylcholine receptor (hM3D) introduced into a cell in a brain of a live primate subject,
the substance being a compound represented by Formula (IV) or a pharmaceutically acceptable salt or solvate thereof: 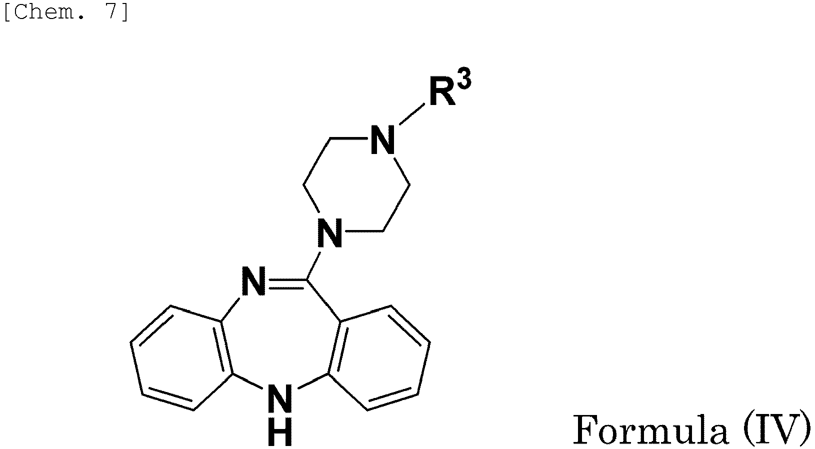
wherein R³ represents hydrogen, alkyl, allyl, hydroxyalkyl, alkoxyalkyl, or alkoyloxyalkyl;
the alkyl having 1 to 6 carbon atoms, the hydroxyalkyl having 1 to 2 carbon atoms, and the alkoxyalkyl and the alkoyloxyalkyl having 1 to 5 carbon atoms.

14. A companion diagnostic agent comprising:
a substance that selectively binds to a mutated human M4 muscarinic acetylcholine receptor (hM4D) or a mutated human M3 muscarinic acetylcholine receptor (hM3D) introduced into a cell in a brain of a live primate subject,
the substance being a compound represented by Formula (IV) or a pharmaceutically acceptable salt or solvate thereof: 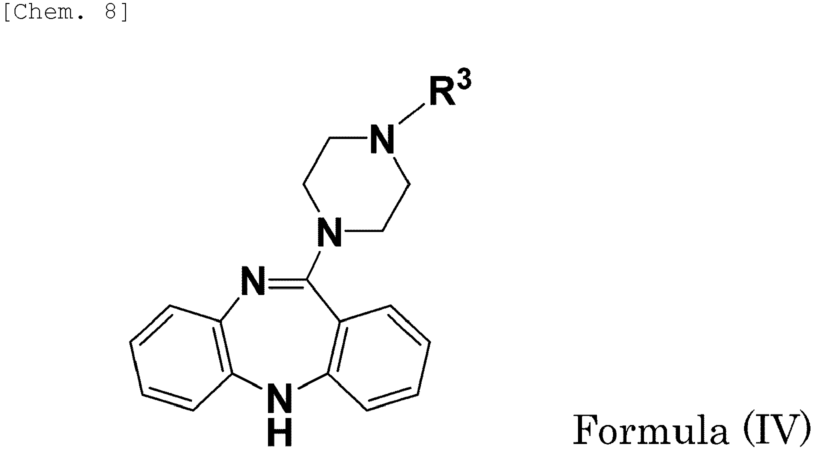
wherein R³ represents hydrogen, alkyl, allyl, hydroxyalkyl, alkoxyalkyl, or alkoyloxyalkyl;
the alkyl having 1 to 6 carbon atoms, the hydroxyalkyl having 1 to 2 carbon atoms,
and the alkoxyalkyl and the alkoyloxyalkyl having 1 to 5 carbon atoms;
wherein one or more atoms are or are not radioisotopes of the atom or atoms.

15. A method for imaging a nerve cell across a plurality of regions in a brain of a live animal subject,
the nerve cell including a dendrite-containing body belonging to a first region and having axon terminals belonging to a region different from the first region,
the first region having an introduced gene encoding a mutated human M4 muscarinic acetylcholine receptor (hM4D) or a mutated human M3 muscarinic acetylcholine receptor (hM3D), the method comprising:
aquiring data on a distribution and/or an amount of expression of the hM4D or the hM3D in the axon terminal in the live amimal subject administred with a radiolabeled dibenzoazepine derivative that is allowed to migrate into the brain to selectively bind to the hM4D or the hM3D, wherein
the data is acquired by detecting radiation emitted from the radiolabeled dibenzoazepine derivative selectively binding to the hM4D or the hM3D.

16. A composition for imaging axon terminals of a nerve cell having an introduced gene encoding a mutated human M4 muscarinic acetylcholine receptor (hM4D) or a mutated human M3 muscarinic acetylcholine receptor (hM3D) in a brain of a live animal subject, the composition comprising:
a radiolabeled dibenzoazepine derivative or a pharmaceutically acceptable salt or solvate thereof.


SEQUENCE LISTING

<110>  National Institutes for Quantum and Radiological Science and
Technology

<120>  Novel Compound binding to Designer Receptor, Imaging Method of
Designer Receptor, Agonist or Antagonist, Therapeutic Agent, Companion
Diagnostic Agent and Imaging Method of Neuron

<130>  AT1-2327

<160>  6

<170>  PatentIn version 3.5

<210>  1
<211>  479
<212>  PRT
<213>  Homo sapiens

<400>  1

Met Ala Asn Phe Thr Pro Val Asn Gly Ser Ser Gly Asn Gln Ser Val
1               5                   10                  15

Arg Leu Val Thr Ser Ser Ser His Asn Arg Tyr Glu Thr Val Glu Met
            20                  25                  30

Val Phe Ile Ala Thr Val Thr Gly Ser Leu Ser Leu Val Thr Val Val
        35                  40                  45

Gly Asn Ile Leu Val Met Leu Ser Ile Lys Val Asn Arg Gln Leu Gln
    50                  55                  60

Thr Val Asn Asn Tyr Phe Leu Phe Ser Leu Ala Cys Ala Asp Leu Ile
65                  70                  75                  80

Ile Gly Ala Phe Ser Met Asn Leu Tyr Thr Val Tyr Ile Ile Lys Gly
                85                  90                  95

Tyr Trp Pro Leu Gly Ala Val Val Cys Asp Leu Trp Leu Ala Leu Asp
            100                 105                 110

Tyr Val Val Ser Asn Ala Ser Val Met Asn Leu Leu Ile Ile Ser Phe
        115                 120                 125

Asp Arg Tyr Phe Cys Val Thr Lys Pro Leu Thr Tyr Pro Ala Arg Arg
    130                 135                 140

Thr Thr Lys Met Ala Gly Leu Met Ile Ala Ala Ala Trp Val Leu Ser
145                 150                 155                 160

Phe Val Leu Trp Ala Pro Ala Ile Leu Phe Trp Gln Phe Val Val Gly

                165                 170                 175

Lys Arg Thr Val Pro Asp Asn Gln Cys Phe Ile Gln Phe Leu Ser Asn
            180                 185                 190

Pro Ala Val Thr Phe Gly Thr Ala Ile Ala Ala Phe Tyr Leu Pro Val
        195                 200                 205

Val Ile Met Thr Val Leu Tyr Ile His Ile Ser Leu Ala Ser Arg Ser
    210                 215                 220

Arg Val His Lys His Arg Pro Glu Gly Pro Lys Glu Lys Lys Ala Lys
225                 230                 235                 240

Thr Leu Ala Phe Leu Lys Ser Pro Leu Met Lys Gln Ser Val Lys Lys
                245                 250                 255

Pro Pro Pro Gly Glu Ala Ala Arg Glu Glu Leu Arg Asn Gly Lys Leu
            260                 265                 270

Glu Glu Ala Pro Pro Pro Ala Leu Pro Pro Pro Pro Arg Pro Val Ala
        275                 280                 285

Asp Lys Asp Thr Ser Asn Glu Ser Ser Ser Gly Ser Ala Thr Gln Asn
    290                 295                 300

Thr Lys Glu Arg Pro Ala Thr Glu Leu Ser Thr Thr Glu Ala Thr Thr
305                 310                 315                 320

Pro Ala Met Pro Ala Pro Pro Leu Gln Pro Arg Ala Leu Asn Pro Ala
                325                 330                 335

Ser Arg Trp Ser Lys Ile Gln Ile Val Thr Lys Gln Thr Gly Asn Glu
            340                 345                 350

Cys Val Thr Ala Ile Glu Ile Val Pro Ala Thr Pro Ala Gly Met Arg
        355                 360                 365

Pro Ala Ala Asn Val Ala Arg Lys Phe Ala Ser Ile Ala Arg Asn Gln
    370                 375                 380

Val Arg Lys Lys Arg Gln Met Ala Ala Arg Glu Arg Lys Val Thr Arg
385                 390                 395                 400

Thr Ile Phe Ala Ile Leu Leu Ala Phe Ile Leu Thr Trp Thr Pro Tyr
                405                 410                 415

Asn Val Met Val Leu Val Asn Thr Phe Cys Gln Ser Cys Ile Pro Asp
            420                 425                 430

Thr Val Trp Ser Ile Gly Tyr Trp Leu Cys Tyr Val Asn Ser Thr Ile
        435                 440                 445

Asn Pro Ala Cys Tyr Ala Leu Cys Asn Ala Thr Phe Lys Lys Thr Phe
    450                 455                 460

Arg His Leu Leu Leu Cys Gln Tyr Arg Asn Ile Gly Thr Ala Arg
465                 470                 475

<210>  2
<211>  479
<212>  PRT
<213>  Homo sapiens

<400>  2

Met Ala Asn Phe Thr Pro Val Asn Gly Ser Ser Gly Asn Gln Ser Val
1               5                   10                  15

Arg Leu Val Thr Ser Ser Ser His Asn Arg Tyr Glu Thr Val Glu Met
            20                  25                  30

Val Phe Ile Ala Thr Val Thr Gly Ser Leu Ser Leu Val Thr Val Val
        35                  40                  45

Gly Asn Ile Leu Val Met Leu Ser Ile Lys Val Asn Arg Gln Leu Gln
    50                  55                  60

Thr Val Asn Asn Tyr Phe Leu Phe Ser Leu Ala Cys Ala Asp Leu Ile
65                  70                  75                  80

Ile Gly Ala Phe Ser Met Asn Leu Tyr Thr Val Tyr Ile Ile Lys Gly
                85                  90                  95

Tyr Trp Pro Leu Gly Ala Val Val Cys Asp Leu Trp Leu Ala Leu Asp
            100                 105                 110

Cys Val Val Ser Asn Ala Ser Val Met Asn Leu Leu Ile Ile Ser Phe
        115                 120                 125

Asp Arg Tyr Phe Cys Val Thr Lys Pro Leu Thr Tyr Pro Ala Arg Arg
    130                 135                 140

Thr Thr Lys Met Ala Gly Leu Met Ile Ala Ala Ala Trp Val Leu Ser
145                 150                 155                 160

Phe Val Leu Trp Ala Pro Ala Ile Leu Phe Trp Gln Phe Val Val Gly
                165                 170                 175

Lys Arg Thr Val Pro Asp Asn Gln Cys Phe Ile Gln Phe Leu Ser Asn
            180                 185                 190

Pro Ala Val Thr Phe Gly Thr Ala Ile Ala Gly Phe Tyr Leu Pro Val
        195                 200                 205

Val Ile Met Thr Val Leu Tyr Ile His Ile Ser Leu Ala Ser Arg Ser
    210                 215                 220

Arg Val His Lys His Arg Pro Glu Gly Pro Lys Glu Lys Lys Ala Lys
225                 230                 235                 240

Thr Leu Ala Phe Leu Lys Ser Pro Leu Met Lys Gln Ser Val Lys Lys
                245                 250                 255

Pro Pro Pro Gly Glu Ala Ala Arg Glu Glu Leu Arg Asn Gly Lys Leu
            260                 265                 270

Glu Glu Ala Pro Pro Pro Ala Leu Pro Pro Pro Pro Arg Pro Val Ala
        275                 280                 285

Asp Lys Asp Thr Ser Asn Glu Ser Ser Ser Gly Ser Ala Thr Gln Asn
    290                 295                 300

Thr Lys Glu Arg Pro Ala Thr Glu Leu Ser Thr Thr Glu Ala Thr Thr
305                 310                 315                 320

Pro Ala Met Pro Ala Pro Pro Leu Gln Pro Arg Ala Leu Asn Pro Ala
                325                 330                 335

Ser Arg Trp Ser Lys Ile Gln Ile Val Thr Lys Gln Thr Gly Asn Glu
            340                 345                 350

Cys Val Thr Ala Ile Glu Ile Val Pro Ala Thr Pro Ala Gly Met Arg
        355                 360                 365

Pro Ala Ala Asn Val Ala Arg Lys Phe Ala Ser Ile Ala Arg Asn Gln
    370                 375                 380

Val Arg Lys Lys Arg Gln Met Ala Ala Arg Glu Arg Lys Val Thr Arg
385                 390                 395                 400

Thr Ile Phe Ala Ile Leu Leu Ala Phe Ile Leu Thr Trp Thr Pro Tyr
                405                 410                 415

Asn Val Met Val Leu Val Asn Thr Phe Cys Gln Ser Cys Ile Pro Asp
            420                 425                 430

Thr Val Trp Ser Ile Gly Tyr Trp Leu Cys Tyr Val Asn Ser Thr Ile
        435                 440                 445

Asn Pro Ala Cys Tyr Ala Leu Cys Asn Ala Thr Phe Lys Lys Thr Phe
    450                 455                 460

Arg His Leu Leu Leu Cys Gln Tyr Arg Asn Ile Gly Thr Ala Arg
465                 470                 475

<210>  3
<211>  1440
<212>  DNA
<213>  Homo sapiens

<400>  3
atggccaact tcacacctgt caatggcagc tcgggcaatc agtccgtgcg cctggtcacg      60

tcatcatccc acaatcgcta tgagacggtg gaaatggtct tcattgccac agtgacaggc     120

tccctgagcc tggtgactgt cgtgggcaac atcctggtga tgctgtccat caaggtcaac     180

aggcagctgc agacagtcaa caactacttc ctcttcagcc tggcgtgtgc tgatctcatc     240

ataggcgcct tctccatgaa cctctacacc gtgtacatca tcaagggcta ctggcccctg     300

ggcgccgtgg tctgcgacct gtggctggcc ctggactgcg tggtgagcaa cgcctccgtc     360

atgaaccttc tcatcatcag ctttgaccgc tacttctgcg tcaccaagcc tctcacctac     420

cctgcccggc gcaccaccaa gatggcaggc ctcatgattg ctgctgcctg ggtactgtcc     480

ttcgtgctct gggcgcctgc catcttgttc tggcagtttg tggtgggtaa gcggacggtg     540

cccgacaacc agtgcttcat ccagttcctg tccaacccag cagtgacctt tggcacagcc     600

attgctggct tctacctgcc tgtggtcatc atgacggtgc tgtacatcca catctccctg     660

gccagtcgca gccgagtcca caagcaccgg cccgagggcc cgaaggagaa gaaagccaag     720

acgctggcct tcctcaagag cccactaatg aagcagagcg tcaagaagcc cccgcccggg     780

gaggccgccc gggaggagct gcgcaatggc aagctggagg aggccccccc gccagcgctg     840

ccaccgccac cgcgccccgt ggctgataag gacacttcca atgagtccag ctcaggcagt     900

gccacccaga acaccaagga acgcccagcc acagagctgt ccaccacaga ggccaccacg     960

cccgccatgc ccgcccctcc cctgcagccg cgggccctca acccagcctc cagatggtcc    1020

aagatccaga ttgtgacgaa gcagacaggc aatgagtgtg tgacagccat tgagattgtg    1080

cctgccacgc cggctggcat gcgccctgcg gccaacgtgg cccgcaagtt cgccagcatc    1140

gctcgcaacc aggtgcgcaa gaagcggcag atggcggccc gggagcgcaa agtgacacga    1200

acgatctttg ccattctgct agccttcatc ctcacctgga cgccctacaa cgtcatggtc 1260

ctggtgaaca ccttctgcca gagctgcatc cctgacacgg tgtggtccat tggctactgg 1320

ctctgctacg tcaacagcac catcaaccct gcctgctatg ctctgtgcaa cgccaccttt 1380

aaaaagacct tccggcacct gctgctgtgc cagtatcgga acatcggcac tgccaggtag 1440

<210> 4
<211> 590
<212> PRT
<213> Homo sapiens

<400> 4

Met Thr Leu His Asn Asn Ser Thr Thr Ser Pro Leu Phe Pro Asn Ile
1               5                   10                  15

Ser Ser Ser Trp Ile His Ser Pro Ser Asp Ala Gly Leu Pro Pro Gly
            20                  25                  30

Thr Val Thr His Phe Gly Ser Tyr Asn Val Ser Arg Ala Ala Gly Asn
        35                  40                  45

Phe Ser Ser Pro Asp Gly Thr Thr Asp Asp Pro Leu Gly Gly His Thr
    50                  55                  60

Val Trp Gln Val Val Phe Ile Ala Phe Leu Thr Gly Ile Leu Ala Leu
65                  70                  75                  80

Val Thr Ile Ile Gly Asn Ile Leu Val Ile Val Ser Phe Lys Val Asn
                85                  90                  95

Lys Gln Leu Lys Thr Val Asn Asn Tyr Phe Leu Leu Ser Leu Ala Cys
            100                 105                 110

Ala Asp Leu Ile Ile Gly Val Ile Ser Met Asn Leu Phe Thr Thr Tyr
        115                 120                 125

Ile Ile Met Asn Arg Trp Ala Leu Gly Asn Leu Ala Cys Asp Leu Trp
    130                 135                 140

Leu Ala Ile Asp Tyr Val Ala Ser Asn Ala Ser Val Met Asn Leu Leu
145                 150                 155                 160

Val Ile Ser Phe Asp Arg Tyr Phe Ser Ile Thr Arg Pro Leu Thr Tyr
                165                 170                 175

Arg Ala Lys Arg Thr Thr Lys Arg Ala Gly Val Met Ile Gly Leu Ala
            180                 185                 190

Trp Val Ile Ser Phe Val Leu Trp Ala Pro Ala Ile Leu Phe Trp Gln
        195                 200                 205

Tyr Phe Val Gly Lys Arg Thr Val Pro Pro Gly Glu Cys Phe Ile Gln
    210                 215                 220

Phe Leu Ser Glu Pro Thr Ile Thr Phe Gly Thr Ala Ile Ala Ala Phe
225                 230                 235                 240

Tyr Met Pro Val Thr Ile Met Thr Ile Leu Tyr Trp Arg Ile Tyr Lys
                245                 250                 255

Glu Thr Glu Lys Arg Thr Lys Glu Leu Ala Gly Leu Gln Ala Ser Gly
            260                 265                 270

Thr Glu Ala Glu Thr Glu Asn Phe Val His Pro Thr Gly Ser Ser Arg
        275                 280                 285

Ser Cys Ser Ser Tyr Glu Leu Gln Gln Gln Ser Met Lys Arg Ser Asn
    290                 295                 300

Arg Arg Lys Tyr Gly Arg Cys His Phe Trp Phe Thr Thr Lys Ser Trp
305                 310                 315                 320

Lys Pro Ser Ser Glu Gln Met Asp Gln Asp His Ser Ser Ser Asp Ser
                325                 330                 335

Trp Asn Asn Asn Asp Ala Ala Ala Ser Leu Glu Asn Ser Ala Ser Ser
            340                 345                 350

Asp Glu Glu Asp Ile Gly Ser Glu Thr Arg Ala Ile Tyr Ser Ile Val
        355                 360                 365

Leu Lys Leu Pro Gly His Ser Thr Ile Leu Asn Ser Thr Lys Leu Pro
    370                 375                 380

Ser Ser Asp Asn Leu Gln Val Pro Glu Glu Glu Leu Gly Met Val Asp
385                 390                 395                 400

Leu Glu Arg Lys Ala Asp Lys Leu Gln Ala Gln Lys Ser Val Asp Asp
                405                 410                 415

Gly Gly Ser Phe Pro Lys Ser Phe Ser Lys Leu Pro Ile Gln Leu Glu
            420                 425                 430

Ser Ala Val Asp Thr Ala Lys Thr Ser Asp Val Asn Ser Ser Val Gly
        435                 440                 445

Lys Ser Thr Ala Thr Leu Pro Leu Ser Phe Lys Glu Ala Thr Leu Ala
    450                 455                 460

Lys Arg Phe Ala Leu Lys Thr Arg Ser Gln Ile Thr Lys Arg Lys Arg
465                 470                 475                 480

Met Ser Leu Val Lys Glu Lys Lys Ala Ala Gln Thr Leu Ser Ala Ile
                485                 490                 495

Leu Leu Ala Phe Ile Ile Thr Trp Thr Pro Tyr Asn Ile Met Val Leu
            500                 505                 510

Val Asn Thr Phe Cys Asp Ser Cys Ile Pro Lys Thr Phe Trp Asn Leu
        515                 520                 525

Gly Tyr Trp Leu Cys Tyr Ile Asn Ser Thr Val Asn Pro Val Cys Tyr
    530                 535                 540

Ala Leu Cys Asn Lys Thr Phe Arg Thr Thr Phe Lys Met Leu Leu Leu
545                 550                 555                 560

Cys Gln Cys Asp Lys Lys Lys Arg Arg Lys Gln Gln Tyr Gln Gln Arg
                565                 570                 575

Gln Ser Val Ile Phe His Lys Arg Ala Pro Glu Gln Ala Leu
            580                 585                 590

<210>  5
<211>  590
<212>  PRT
<213>  Homo sapiens

<400>  5

Met Thr Leu His Asn Asn Ser Thr Thr Ser Pro Leu Phe Pro Asn Ile
1               5                   10                  15

Ser Ser Ser Trp Ile His Ser Pro Ser Asp Ala Gly Leu Pro Pro Gly
            20                  25                  30

Thr Val Thr His Phe Gly Ser Tyr Asn Val Ser Arg Ala Ala Gly Asn
        35                  40                  45

Phe Ser Ser Pro Asp Gly Thr Thr Asp Asp Pro Leu Gly Gly His Thr
    50                  55                  60

Val Trp Gln Val Val Phe Ile Ala Phe Leu Thr Gly Ile Leu Ala Leu
65                  70                  75                  80

Val Thr Ile Ile Gly Asn Ile Leu Val Ile Val Ser Phe Lys Val Asn
                85                  90                  95

Lys Gln Leu Lys Thr Val Asn Asn Tyr Phe Leu Leu Ser Leu Ala Cys
            100                 105                 110

Ala Asp Leu Ile Ile Gly Val Ile Ser Met Asn Leu Phe Thr Thr Tyr
        115                 120                 125

Ile Ile Met Asn Arg Trp Ala Leu Gly Asn Leu Ala Cys Asp Leu Trp
    130                 135                 140

Leu Ala Ile Asp Cys Val Ala Ser Asn Ala Ser Val Met Asn Leu Leu
145                 150                 155                 160

Val Ile Ser Phe Asp Arg Tyr Phe Ser Ile Thr Arg Pro Leu Thr Tyr
                165                 170                 175

Arg Ala Lys Arg Thr Thr Lys Arg Ala Gly Val Met Ile Gly Leu Ala
            180                 185                 190

Trp Val Ile Ser Phe Val Leu Trp Ala Pro Ala Ile Leu Phe Trp Gln
        195                 200                 205

Tyr Phe Val Gly Lys Arg Thr Val Pro Pro Gly Glu Cys Phe Ile Gln
    210                 215                 220

Phe Leu Ser Glu Pro Thr Ile Thr Phe Gly Thr Ala Ile Ala Gly Phe
225                 230                 235                 240

Tyr Met Pro Val Thr Ile Met Thr Ile Leu Tyr Trp Arg Ile Tyr Lys
                245                 250                 255

Glu Thr Glu Lys Arg Thr Lys Glu Leu Ala Gly Leu Gln Ala Ser Gly
            260                 265                 270

Thr Glu Ala Glu Thr Glu Asn Phe Val His Pro Thr Gly Ser Ser Arg
        275                 280                 285

Ser Cys Ser Ser Tyr Glu Leu Gln Gln Gln Ser Met Lys Arg Ser Asn
    290                 295                 300

Arg Arg Lys Tyr Gly Arg Cys His Phe Trp Phe Thr Thr Lys Ser Trp
305                 310                 315                 320

Lys Pro Ser Ser Glu Gln Met Asp Gln Asp His Ser Ser Ser Asp Ser

                325                 330                 335

Trp Asn Asn Asn Asp Ala Ala Ala Ser Leu Glu Asn Ser Ala Ser Ser
            340                 345                 350

Asp Glu Glu Asp Ile Gly Ser Glu Thr Arg Ala Ile Tyr Ser Ile Val
        355                 360                 365

Leu Lys Leu Pro Gly His Ser Thr Ile Leu Asn Ser Thr Lys Leu Pro
    370                 375                 380

Ser Ser Asp Asn Leu Gln Val Pro Glu Glu Glu Leu Gly Met Val Asp
385                 390                 395                 400

Leu Glu Arg Lys Ala Asp Lys Leu Gln Ala Gln Lys Ser Val Asp Asp
                405                 410                 415

Gly Gly Ser Phe Pro Lys Ser Phe Ser Lys Leu Pro Ile Gln Leu Glu
            420                 425                 430

Ser Ala Val Asp Thr Ala Lys Thr Ser Asp Val Asn Ser Ser Val Gly
        435                 440                 445

Lys Ser Thr Ala Thr Leu Pro Leu Ser Phe Lys Glu Ala Thr Leu Ala
    450                 455                 460

Lys Arg Phe Ala Leu Lys Thr Arg Ser Gln Ile Thr Lys Arg Lys Arg
465                 470                 475                 480

Met Ser Leu Val Lys Glu Lys Lys Ala Ala Gln Thr Leu Ser Ala Ile
                485                 490                 495

Leu Leu Ala Phe Ile Ile Thr Trp Thr Pro Tyr Asn Ile Met Val Leu
            500                 505                 510

Val Asn Thr Phe Cys Asp Ser Cys Ile Pro Lys Thr Phe Trp Asn Leu
        515                 520                 525

Gly Tyr Trp Leu Cys Tyr Ile Asn Ser Thr Val Asn Pro Val Cys Tyr
    530                 535                 540

Ala Leu Cys Asn Lys Thr Phe Arg Thr Thr Phe Lys Met Leu Leu Leu
545                 550                 555                 560

Cys Gln Cys Asp Lys Lys Lys Arg Arg Lys Gln Gln Tyr Gln Gln Arg
                565                 570                 575

Gln Ser Val Ile Phe His Lys Arg Ala Pro Glu Gln Ala Leu
            580                 585                 590

<210>  6
<211>  1773
<212>  DNA
<213>  Homo sapiens

<400>  6
atgaccttgc acaataacag tacaacctcg cctttgtttc caaacatcag ctcctcctgg      60

atacacagcc cctccgatgc agggctgccc ccgggaaccg tcactcattt cggcagctac     120

aatgtttctc gagcagctgg caatttctcc tctccagacg gtaccaccga tgaccctctg     180

ggaggtcata ccgtctggca agtggtcttc atcgctttct taacgggcat cctggccttg     240

gtgaccatca tcggcaacat cctggtaatt gtgtcattta aggtcaacaa gcagctgaag     300

acggtcaaca actacttcct cttaagcctg gcctgtgccg atctgattat cggggtcatt     360

tcaatgaatc tgtttacgac ctacatcatc atgaatcgat gggccttagg gaacttggcc     420

tgtgacctct ggcttgccat tgactacgta gccagcaatg cctctgttat gaatcttctg     480

gtcatcagct ttgacagata cttttccatc acgaggccgc tcacgtaccg agccaaacga     540

acaacaaaga gagccggtgt gatgatcggt ctggcttggg tcatctcctt tgtcctttgg     600

gctcctgcca tcttgttctg gcaatacttt gttggaaaga gaactgtgcc tccgggagag     660

tgcttcattc agttcctcag tgagcccacc attacttttg gcacagccat cgctggtttt     720

tatatgcctg tcaccattat gactatttta tactggagga tctataagga aactgaaaag     780

cgtaccaaag agcttgctgg cctgcaagcc tctgggacag aggcagagac agaaaacttt     840

gtccacccca cgggcagttc tcgaagctgc agcagttacg aacttcaaca gcaaagcatg     900

aaacgctcca acaggaggaa gtatggccgc tgccacttct ggttcacaac caagagctgg     960

aaacccagct ccgagcagat ggaccaagac cacagcagca gtgacagttg gaacaacaat    1020

gatgctgctg cctccctgga gaactccgcc tcctccgacg aggaggacat tggctccgag    1080

acgagagcca tctactccat cgtgctcaag cttccgggtc acagcaccat cctcaactcc    1140

accaagttac cctcatcgga caacctgcag gtgcctgagg aggagctggg gatggtggac    1200

ttggagagga aagccgacaa gctgcaggcc cagaagagcg tggacgatgg aggcagtttt    1260

ccaaaaagct tctccaagct tcccatccag ctagagtcag ccgtggacac agctaagact    1320

tctgacgtca actcctcagt gggtaagagc acggccactc tacctctgtc cttcaaggaa    1380

gccactctgg ccaagaggtt tgctctgaag accagaagtc agatcactaa gcggaaaagg    1440

atgtccctgg tcaaggagaa gaaagcggcc cagaccctca gtgcgatctt gcttgccttc    1500

atcatcactt ggaccccata caacatcatg gttctggtga acaccttttg tgacagctgc    1560

atacccaaaa ccttttggaa tctgggctac tggctgtgct acatcaacag caccgtgaac    1620

cccgtgtgct atgctctgtg caacaaaaca ttcagaacca ctttcaagat gctgctgctg 1680

tgccagtgtg acaaaaaaaa gaggcgcaag cagcagtacc agcagagaca gtcggtcatt 1740

tttcacaagc gcgcacccga gcaggccttg tag 1773